# EUROPEAN PATENT SPECIFICATION

(11) **EP 1 379 505 B1**
(45) Date of publication and mention of the grant of the patent: **28.02.2007**
(21) Application number: 02725710.4
(22) Date of filing: 18.04.2002
(51) Int. Cl.: C07D 213/40, C07D 401/12, C07D 215/38, A61K 31/44, A61K 31/4709, A61K 31/4725, A61K 31/47, A61P 43/00

(54) **INHIBITION OF RAF KINASE USING QUINOLYL, ISOQUINOLYL OR PYRIDYL UREAS**
INHIBIERUNG DER RAF-KINASE DURCH CHINOLIN-, ISOCHINOLIN- ODER PYRIDIN-HARNSTOFFE
INHIBITION DE LA KINASE RAF A L'AIDE D'UREES DE QUINOLYL, D'ISOQUINOLYL OU DE PYRIDYL

(30) Priority: 20.04.2001 US 838285
(43) Date of publication of application: 14.01.2004
(73) Proprietor: Bayer Pharmaceuticals Corporation, West Haven, CT 06516 (US)
(72) Inventor: DUMAS, Jacques, Bethany, CT 06524 (US); RIEDL, Bernd, D-42329 Wuppertal (DE); KHIRE, Uday, Hamden, CT 06518 (US); SIBLEY, Robert, N., North Haven, CT 06437 (US); HATOUM-MOKDAD, Holia, Hamden, CT 06514 (US); MONAHAN, Mary-Katherine, Hamden, CT 06517 (US); GUNN, David, E., Hamden, CT 06517 (US); LOWINGER, Timothy, B., D-42117 Wuppertal (DE); SCOTT, William, J., Guilford, CT 06437 (US); SMITH, Roger, A., Madison, CT 06443 (US); WOOD, Jill, E., North Haven, CT 06473 (US)
(74) Representative: Portal, Gérard
(86) International application number: PCT/US2002/012066
(87) International publication number: WO 2002/085857

(56) References cited:
- WO-A-00/41698
- WO-A-00/42012
- WO-A-00/47577
- WO-A-02/24635
- WO-A-94/18170
- WO-A-99/21835
- WO-A-99/24398
- WO-A-99/32106
- WO-A-99/32436
- WO-A-02/062763
- US-A- 5 596 001
- US-A1- 2002 065 296

## Description

### Field of the Invention

This invention relates to the use of a group of aryl ureas in treating raf mediated diseases, and pharmaceutical compositions for use in such therapy.

### Background of the Invention

The p21^{ras} oncogene is a major contributor to the development and progression of human solid cancers and is mutated in 30% of all human cancers (Bolton et al. *Ann. Rep. Med. Chem.* **1994**, 29, 165-74; Bos. *Cancer Res.* **1989**, 49, 4682-9). In its normal, unmutated form, the ras protein is a key element of the signal transduction cascade directed by growth factor receptors in almost all tissues (Avruch et al. *Trends Biochem. Sci.* **1994**, 19, 279-83). Biochemically, ras is a guanine nucleotide binding protein, and cycling between a GTP-bound activated and a GDP-bound resting form is strictly controlled by ras' endogenous GTPase activity and other regulatory proteins. In the ras mutants in cancer cells, the endogenous GTPase activity is alleviated and, therefore, the protein delivers constitutive growth signals to downstream effectors such as the enzyme raf kinase. This leads to the cancerous growth of the cells which carry these mutants (Magnuson et al. *Semin. Cancer Biol*. **1994,** *5*, 247-53). It has been shown that inhibiting the effect of active ras by inhibiting the raf kinase signaling pathway by administration of deactivating antibodies to raf kinase or by co-expression of dominant negative raf kinase or dominant negative MEK, the substrate of raf kinase, leads to the reversion of transformed cells to the normal growth phenotype (see: Daum et al. *Trends Biochem. Sci.* **1994,** *19,* 474-80; Fridman et al. *J. Biol. Chem.* **1994**, *269,* 30105-8. Kolch et al. (*Nature* **1991**, *349,* 426-28) have further indicated that inhibition of raf expression by antisense RNA blocks cell proliferation in membrane-associated oncogenes. Similarly, inhibition of raf kinase (by antisense oligodeoxynucleotides) has been correlated in vitro and in vivo with inhibition of the growth of a variety of human tumor types (Monia et al., *Nat. Med*. **1996**, *2*, 668-75).

WO 99/32436, WO 99/32106 and WO 0042012 teach the use of a group of aryl ureas in treating raf mediated diseases and pharmaceutical compositions for use in such therapy. However, none of these documents is directed to the specific class of aryl ureas of formula II herein having an unsubstituted isoquinolinyl or quinolinyl or substituted isoquinolinyl group and a bridged cyclic structure and no example is directed to such specific compounds which are useful for the treatment of a condition where inhibition of the raf kinase pathway is needed.

### Summary of the Invention

The present invention provides compounds which are inhibitors of the enzyme raf kinase. Since the enzyme is a downstream effector of p21^{ras}, the instant inhibitors are useful in pharmaceutical compositions for human or veterinary use where inhibition of the raf kinase pathway is indicated, e.g., in the treatment of tumors and/or cancerous cell growth mediated by raf kinase. In particular, the compounds are useful in the treatment of human or animal, e.g., murine cancer, since the progression of these cancers is dependent upon the ras protein signal transduction cascade and therefore susceptible to treatment by interruption of the cascade, i.e., by inhibiting raf kinase. Accordingly, the compounds of the invention are useful in treating solid cancers, such as, for example, carcinomas (e.g., of the lungs, pancreas, thyroid, bladder or colon, myeloid disorders (e.g., myeloid leukemia) or adenomas (e.g., villous colon adenoma).

The present invention, therefore, provides compounds generally described as aryl ureas, including both aryl and heteroaryl analogues, which inhibit the raf pathway. The invention also provides a method for treating a raf mediated disease state in humans or mammals. Thus, the invention is directed to compounds which inhibit the enzyme RAF kinase and also to compounds, compositions and methods for the treatment of cancerous cell growth mediated by raf kinase wherein a compound of the formula II or a pharmaceutically acceptable salt thereof, is administered.

A' - D - B' (II)

In formula II,
D is -NH-C(O)-NH-,
A' is a substituted isoquinolinyl group or unsubstituted isoquinolinyl group or an unsubstituted quinolinyl group,
B' is a substituted or unsubstituted bridged cyclic structure of up to 30 carbon atoms of the formula -L-(ML¹)_{q} wherein L comprises a cyclic moiety having at least 5 members and is bound directly to D, L¹ comprises a cyclic moiety having at least 5 members, M is a bridging group having at least one atom, q is an integer of from 1-3, and each cyclic structure of L and L¹ contains 0-4 members of the group consisting of nitrogen, oxygen and sulfur,
subject to the provisos that
B' is not

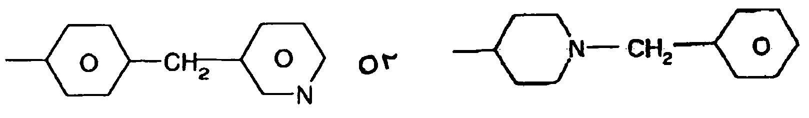

The substituted isoquinolinyl groups of A' are selected from the group consisting of halogen, up to per-halo, and Wn, where n is 0-3 and each W is independently selected from the group consisting of C₁₋₁₀ alkyl, C₁₋₁₀ alkoxy, at least a five membered C₃₋₁₀ cycloalkyl having 0-3 heteroatoms, C₂₋₁₀ alkenyl, C₁₋₁₀ alkenoyl, substituted C₁₋₁₀ alkyl, substituted C₁₋₁₀ alkoxy, a substituted C₃₋₁₀ cycloalkyl having at least 5 cyclic members and 0-3 heteroatoms selected from N, S and O; C₆-C₁₄ aryl, C₇-C₂₄ alkaryl, C₇ -C₂₄ aralkyl, C₃-C₁₂ heteroaryl having 1-3 heteroatoms selected from O, N and S, C₄-C₂₃ alkheteroaryl having 1-3 heteroatoms selected from O, N and S, up to per halo substituted C₆-C₁₂ aryl, up to per halo substituted C₃-C₁₂ hetaryl having at least 5 members and 1-3 heteroatoms selected from O, N and S, up to per halo substituted C₇-C₂₄ aralkyl, up to per halo substituted C₇-C₂₄ alkaryl, up to per halo substituted C₄-C₂₃ alkheteroaryl having at least 5 cyclic members and 1-3 heteroatoms selected from O, N and S, -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl.

Where B' is substituted, the substituents are selected from the group consisting of halogen, up to per-halo, and Jn, where n is 0-3 and each J is independently selected from the group consisting of -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, C₁₋₁₀ alkyl, C₁₋₁₀ alkoxy, C₃₋₁₀ cycloalkyl having at least five cyclic members and 0-3 heteroatoms, C₂₋₁₀ alkenyl, C₁₋₁₀ alkenoyl, C₆₋₁₂ aryl, C₃₋₁₂ hetaryl having at least five cyclic members and 1-3 heteroatoms selected from N, S and O, C₇₋₂₄ aralkyl, C₇₋₂₄ alkaryl, substituted C₁₋₁₀ alkyl, substituted C₁₋₁₀ alkoxy, substituted C₃₋₁₀ cycloalkyl having at least five cyclic members and 0-3 heteroatoms selected from N, S and O, substituted C₆ -C₁₄ aryl, substituted C₃₋₁₂ hetaryl having at least five cyclic members and 1-3 heteroatoms selected from N, S and O, substituted C₇₋₂₄ alkaryl , substituted C₇-C₂₄ aralkyl and -Q-Ar, subject to the proviso that where is -L(ML¹)_{q}, L¹ is not substituted by the substituents -C(O)R^{a}, -C(NR²)R^{b}, -C(O)NR^{a}R^{b} and -SO₂R^{a} wherein R^{a} and R^{b} are each, independently, hydrogen or a carbon based moiety of up to 24 carbon atoms, optionally containing heteroatoms selected from N, S and O.

R^{a} and R^{b} are preferably C₁₋₁₀ alkyl, C₁₋₁₀ alkoxy, C₃₋₁₀ cycloalkyl having 0-3 heteroatoms, C₂₋₁₀ alkenyl, C₁₋₁₀ alkenoyl, C₆₋₁₂ aryl, C₃₋₁₂ hetaryl having 1-3 heteroatoms selected from N, S and O, C₇₋₂₄ aralkyl, C₇₋₂₄ alkaryl, substituted C₁₋₁₀ alkyl, substituted C₁₋₁₀ alkoxy, substituted C₃₋₁₀ cycloalkyl having 0-3 heteroatoms selected from N, S and O, substituted C₆ -C₁₄ aryl, substituted C₃₋₁₂ hetaryl having 1-3 heteroatoms selected from N, S and O, substituted C₇₋₂₄ alkaryl or substituted C₇-C₂₄ aralkyl, where R^{a} is a substituted group, it is substituted by halogen up to per halo.

Where J is a substituted group, it is substituted by halogen, up to per halo, or by one or more substituents independently selected from the group consisting of -CN, -CO₂R⁷, -OR⁷, -SR⁷, -NR⁷R^{7'}, -NO₂, -NR⁷C(O)R^{7'}, -NR⁷C(O)OR^{7'}; with each R⁷ and R^{7'} independently as defined above.

Where the substituents for B' are -Q-Ar, Q is -O-, -S-, -N(R⁷)-, -(CH₂)ₘ-, -C(O)-, -O-[C(R⁹)(R^{9'})]ₘ-, -CH(OH)-, -(CH₂)ₘO-, -(CH₂)ₘS-, -(CH₂)ₘN(R⁷)-, -O(CH₂)ₘ- ,-CHX^{a}-, -CX^{a}₂-, -S-(CH₂)ₘ- and -N(R⁷)(CH₂)ₘ-, where m= 1-3, R⁹ and R^{9'} are each, independently, hydrogen, C₁-C₄ alkyl and halogen, and X^{a} is halogen and each R⁷ is as defined above, and

Ar is a 5- or 6-member aromatic structure. This aromatic structure of Ar
a) contains 0-2 members selected from the group consisting of nitrogen, oxygen and sulfur,
b) is free of the substituents -C(O)R^{a}, -C(NR^{a})R^{b}, -C(O)NR^{a}R^{b}, and -SO₂R^{a}, wherein R^{a} and R^{b} are as defined above;
c) is optionally substituted by halogen, up to per-halo, and
d) is optionally substituted by Mp, wherein p is 0 to 3 and each M is independently selected from the group consisting of -CN, -NO₂, -OR⁷, - SR⁷, -NR⁷R^{7'}, -NR⁷C(O)OR^{7'}, -NR⁷C(O)R⁷', with each R⁷ and R⁷' independently as defined above, C₁₋₁₀ alkyl, C₁₋₁₀ alkoxy, C₂₋₁₀ alkenyl and C₁₋₁₀ alkenoyl halo substituted C₁₋₁₀ alkyl up to per halo, halo substituted C₁₋₁₀ alkoxy up to per halo, halosubstituted C₂₋₁₀ alkenyl up to per halo and halosubstituted C₁₋₁₀ alkenoyl up to per halo.

The bridging group M in the formula -L-(ML¹)_{q}, for is selected from the group consisting of -O-, -S-, -N(R⁷)-, -(CH₂)ₘ-, -C(O)-, -CH(OH)-, -(CH₂)ₘO-, -(CH₂)ₘS-, -( CH₂)ₘ N(R⁷)-, -O(CH₂)ₘ- CHX^{a}-, -CX^{a}₂-, -S-(CH₂)ₘ- and -N(R⁷)(CH₂)ₘ-, where m=1-3, X^{a} is hydrogen and R⁷ is as defined above and q is 1. More preferably, M is -O-, -CH₂-, -S-, -NH-, -C(O)-, -O-CH₂- and -CH₂-O-.

The moieties L and L¹ in the formula -L-(ML¹)_{q} for B' are typically each, independently, a substituted aryl moiety having at least 6 cyclic members, a substituted hetaryl moiety having at least 5 cyclic members, an unsubstituted aryl moiety having at least 6 cyclic members or an unsubstituted hetaryl moiety having at least 5 cyclic members. The hetaryl moietes for L and L' typically have 1 to 4 members selected from the group of hetaryl atoms consisting of nitrogen, oxygen and sulfur with the balance of the hetaryl moiety being carbon. More typical moieties for L¹ and L are selected from the group consisting of thiophene, phenyl, substituted phenyl, pyridinyl, substituted pyridinyl, pyrimidinyl substituted pyrimidinyl, quinolyl, substituted quinolyl isoquinolyl, substituted isoquinolyl, napthyl and substituted napthyl.

The substituted isoquinolinyls of A' preferably have 1-3 substituents selected from the group consisting of C₁₋₁₀ alkyl, up to per halo substituted C₁₋₁₀ alkyl, -CN, -OH, halogen, C₁₋₁₀ alkoxy, up to per halo substituted C₁₋₁₀ alkoxy and C3-C10 heterocyclic moieties comprising 1 to 2 heteroatoms selected from the group of consisting of nitrogen, oxygen and sulfur.

In Formula II suitable hetaryl groups include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms. For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or -5-yl, 1- or 5-tetrazolyl, 1,2,3-oxadiazol-4- or-5-yl, 1,2,4-oxadiazol-3- or -5-yl, 1,3,4-thiadiazol-2- or -5-yl, 1,2,4-oxadiazol-3- or -5-yl, 1,3,4-thiadiazol-2- or -5-yl, 1,3,4-thiadiazol-3- or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8-isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc. For example, B can be 4-methyl-phenyl, 5-methyl-2-thienyl, 4-methyl-2-thienyl, 1-methyl-3-pyrryl, 1-methyl-3-pyrazolyl, 5-methyl-2-thiazolyl or 5-methyl-1,2,4-thiadiazol-2-yl.

Suitable alkyl groups and alkyl portions of groups, e.g., alkoxy, etc. throughout include methyl, ethyl, propyl, butyl, etc., including all straight-chain and branched isomers such as isopropyl, isobutyl, *sec*-butyl, *tert*-butyl, etc.

Suitable aryl groups which do not contain heteroatoms include, for example, phenyl and 1- and 2-naphthyl.

The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl", as used herein also includes saturated heterocyclic groups.

Suitable halogen groups include F, Cl, Br, and/or I, from one to per-substitution (i.e. all H atoms on a group replaced by a halogen atom) being possible where an alkyl group is substituted by halogen, mixed substitution of halogen atom types also being possible on a given moiety.

The invention also relates to compounds *per se,* of formula II.

The present invention is also directed to pharmaceutically acceptable salts of formula II. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, trifluoromethanesulfonic acid, benzenesulphonic acid, *p*-toluenesulfonic acid, 1-naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include acid salts of inorganic bases, such as salts containing alkaline cations (e.g., Li⁺ Na⁺ or K⁺), alkaline earth cations (e.g., Mg⁺² , Ca⁺² or Ba⁺²), the ammonium cation, as well as acid salts of organic bases, including aliphatic and aromatic substituted ammonium, and quaternary ammonium cations, such as those arising from protonation or peralkylation of triethylamine, *N,N*-diethylamine, *N,N*-dicyclohexylamine, lysine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formula II possess asymmetric carbons and can therefore exist in racemic and optically active forms. Methods of separation of enantiomeric and diastereomeric mixtures are well known to one skilled in the art. The present invention encompasses any racemic or optically active form of compounds described in Formula II which possess progesterone receptor binding activity.

### General Preparative Methods

The compounds of Formula II may be prepared by the use of known chemical reactions and procedures, some from starting materials which are commercially available. Nevertheless, general preparative methods are provided below to aid one skilled in the art in synthesizing these compounds, with more detailed examples being provided in the Experimental section which follows.

Substituted and unsubstituted aminoquinolines, aminoisoquinolines and aminopyridines may be prepared using standard methods (see, for example: A.R. Katritzky et al. (Eds.). *Comprehensive Heterocyclic Chemistry II,* Vol. 5. M.H. Palmer. *Heterocyclic Compounds;* Arnold Ltd., London (1967). C.K. Esser et al. WO 96/18616. C.J. Donahue et al. *Inorg. Chem.* 30, *1991,* 1588. E. Cho et al. WO 98/00402. A. Cordi et al. *Bioorg. Med. Chem..* 3, *1995*, 129). In addition, many aminoquinolines, aminoisoquinolines and aminopyridines are commercially available.

Substituted anilines may be generated using standard methods (March. *Advanced Organic Chemistry,* 3^{rd} Ed.; John Wiley: New York (1985). Larock. *Comprehensive Organic Transformations*; VCH Publishers: New York (1989)). As shown in Scheme I, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. *Hydrogenation Methods.;* Academic Press: London, UK (1985)). Nitroaryls may also be directly reduced using a strong hydride source, such as LiAlH₄ (Seyden-Penne. *Reductions by the Alumino- and Borohydrides in Organic Synthesis;* VCH Publishers: New York (1991)), or using a zero valent metal, such as Fe, Sn or Ca, often in acidic media. Many methods exist for the synthesis of nitroaryls (March. *Advanced Organic Chemistry*, 3^{rd} Ed.; John Wiley: New York (1985). Larock. *Comprehensive Organic Transformations;* VCH Publishers: New York (1989)).

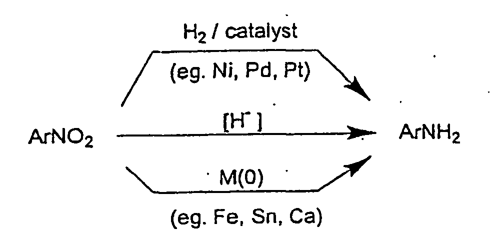

### Scheme I Reduction of Nitroaryls to Aryl Amines

Nitroaryls are commonly formed by electrophilic aromatic nitration using HNO₃, or an alternative NO₂⁺ source. Nitroaryls may be further elaborated prior to reduction. Thus, nitroaryls substituted with 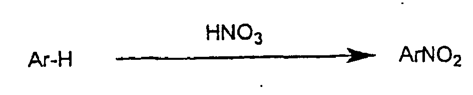 potential leaving groups (eg. F, Cl, Br, etc.) may undergo substitution reactions on treatment with nucleophiles, such as thiolate (exemplified in Scheme II) or phenoxide. Nitroaryls may also undergo Ullman-type coupling reactions (Scheme II).

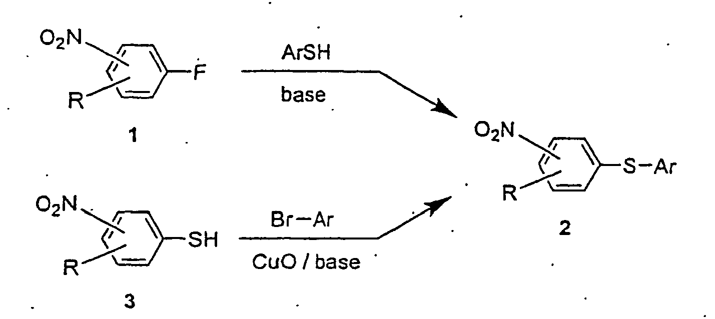

### Scheme II Selected Nucleophilic Aromatic Substitution using Nitroaryls

Nitroaryls may also undergo transition metal mediated cross coupling reactions. For example, nitroaryl electrophiles, such as nitroaryl bromides, iodides or triflates, undergo palladium mediated cross coupling reactions with aryl nucleophiles, such as arylboronic acids (Suzuki reactions, exemplified below), aryltins (Stille reactions) or arylzincs (Negishi reaction) to afford the biaryl **(5)**. 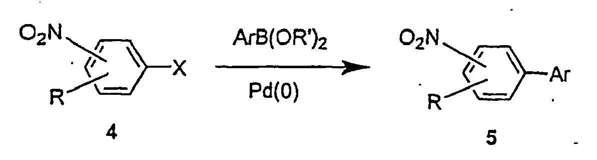 Either nitroaryls or anilines may be converted into the corresponding arenesulfonyl chloride **(7)** on treatment with chlorosulfonic acid. Reaction of the sulfonyl chloride with a fluoride source, such as KF then affords sulfonyl fluoride **(8)**. Reaction of sulfonyl fluoride 8 with trimethylsilyl trifluoromethane in the presence of a fluoride source, such as tris(dimethylamino)sulfonium difluorotrimethylsiliconate (TASF) leads to the corresponding trifluoromethylsulfone **(9)**. Alternatively, sulfonyl chloride 7 may be reduced to the arenethiol **(10)**, for example with zinc amalgum. Reaction of thiol 10 with CHClF₂ in the presence of base gives the difluoromethyl mercaptan **(11)**, which may be oxidized to the sulfone **(12)** with any of a variety of oxidants, including CrO₃-acetic anhydride (Sedova et al. *Zh. Org. Khim.* **1970**, *6,* (568).

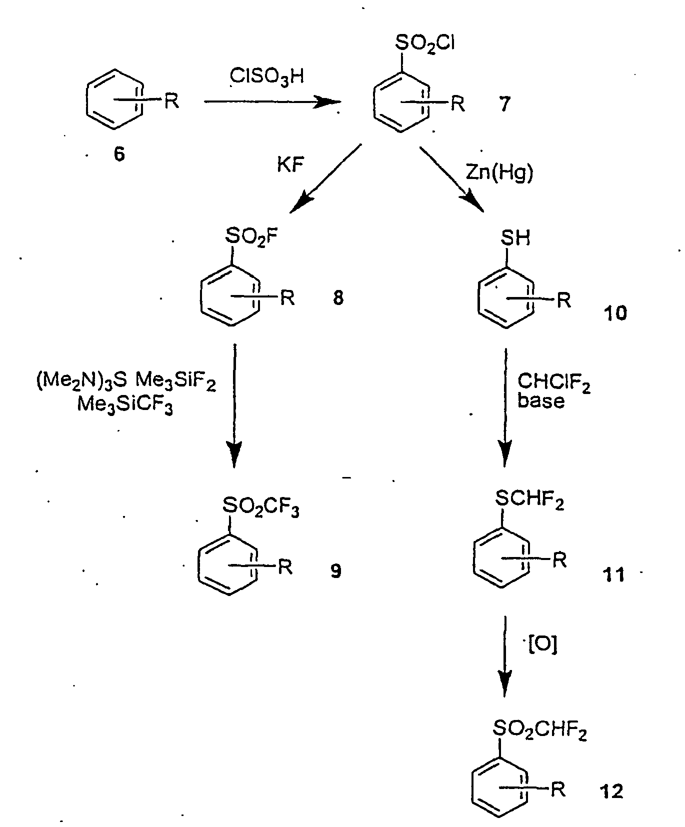

### Scheme III. Selected Methods of Fluorinated Aryl Sulfone Synthesis

As shown in Scheme IV, non-symmetrical urea formation may involve reaction of an aryl isocyanate **(14)** with an aryl amine **(13)**. The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N,N'*-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative 16 with an azide source, followed by rearrangement affords the isocyanate. The corresponding carboxylic acid **(17)** may also be subjected to Curtius-type rearrangements using diphenylphosphoryl azide (DPPA) or a similar reagent.

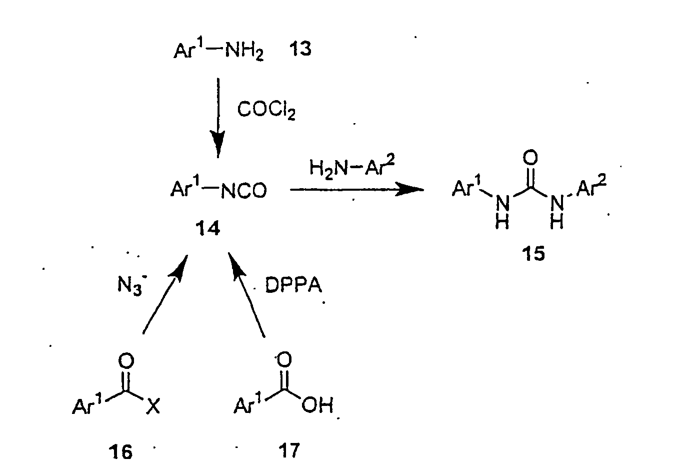

### Scheme IV Selected Methods of Non-Symmetrical Urea Formation

Finally, ureas may be further manipulated using methods familiar to those skilled in the art.

The invention also includes pharmaceutical compositions including at least one compound of Formula I, II or III and a physiologically acceptable carrier.

The compounds may be administered orally, dermally, parenterally, by injection, by inhalation or spray, or sublingually, rectally or vaginally in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl, *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oil phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal or vaginal temperature and will therefore melt in the rectum or vagina to release the drug. Such materials include cocoa butter and polyethylene glycols.

Compounds of the invention may also be administered transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula II may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures one or more materials selected from lower alcohols, lower ketones , lower carboxylic acid esters, polar ethers, lower hydrocarbons, halogenated hydrocarbons.

Suitable penetration enhancing materials for transdermal delivery systems are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, *n*-butyl, *sec*-butyl isobutyl *tert*-butyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated dicarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrene-butadiene coploymers, and natural and synthetic rubbers. Cellulose ethers, derivatized polyethylenes, and silicates may also be used as matrix components. Additional additives, such as viscous resins or oils may be added to increase the viscosity of the matrix.

For all regimens of use disclosed herein for compounds of Formula II, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily rectal dosage regimen will preferably be from 0.01 to 200 mg/Kg of total body weight. The daily vaginal dosage regimen will preferably be from 0.01 to 200 mg/Kg of total body weight. The daily dosages for oral administration, administration by injection, rectal administration and vaginal administration can be achieved by multiple administrations per day or by administration as infrequently as once every 14 days. The long term dosage, can range from 100-800 mg/Kg of total body weight, more preferably 200-600 mg/Kg of total body weight. The daily topical dosage regimen will preferably be from 0.1 to 200 mg administered between one to four times daily. The transdermal concentration will preferably be that required to maintain a daily dose of from 0.01 to 200 mg/Kg of total body weight. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, but not limited to the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, ie., the mode of treatment and the daily or weekly number of doses of a compound of Formula II, or a pharmaceutically acceptable salt thereof given for a defined number of days, can be ascertained by those skilled in the art using conventional treatment tests.

The entire disclosure of all applications, patents and publications cited above and below are hereby incorporated by reference.

The compounds of Formula II are producible from known compounds (or from starting materials which, in turn, are producible from known compounds), e.g., through the general preparative methods shown below. The activity of a given compound to inhibit raf kinase can be routinely assayed, e.g., according to procedures disclosed below. The following examples are for illustrative purposes only and are not intended, nor should they be construed to limit the invention in any way.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg. Unless otherwise-stated, the term 'under high vacuum' refers to a vacuum of 0.4 - 1.0 mmHg.

All temperatures are reported uncorrected in degrees Celsius (°C). Unless otherwise indicated, all parts and percentages are by weight.

Commercial grade reagents and solvents were used without further purification. *N-*cyclohexyl-*N*'-(methylpolystyrene)carbodiimide was purchased from Calbiochem-Novabiochem Corp. 5-(Trifluoromethyl)-2-aminopyridine, 3-aminoqunioline, 3-aminoisoquinoline, 1-(4-methylpiperazinyl)-3-aminoisoquinoline, ethyl 4-isocyanatobenzoate, *N*-acetyl-4-chloro-2-methoxy-5-(trifluoromethyl)aniline, 4-(4-nitrobenzyl)pyridine, 4-phenoxyaniline, 4-(4-methylphenoxy)aniline, 4-(4-chlorophenoxy)aniline and 4-chloro-3-(trifluoromethyl)phenyl isocyanate were purchased and used without further purification. Syntheses of 2-amino-4-*tert*-butylpyridine (C.K. Esser et al. WO 96/18616; C.J. Donahue et al. *Inorg. Chem*. **30**, *1991*, 1588), 3-amino-2-methoxyquinoline (E. Cho et al. WO 98/00402; A. Cordi et al. EP 542,609; *IBID Bioorg. Med. Chem*.. ***3**, 1995,* 129), 4-(3-carbamoylphenoxy)-1-nitrobenzene (K. Ikawa *Yakugaku Zasshi* **79**, *1959,* 760; *Chem. Abstr*. **53**, *1959,* 12761b), 4-[(4-methoxyphenyl)methylamino]aniline (P. Brenneisen et al. US 3,755,406; *IBID* US 3,839,582; *IBID* DE 1,935,388), 4-(4-pyridylcarbonyl)aniline (M.L. Carmello et al. *Pestic. Sci.* **45**, *1995,* 227), 3-*tert*-butylphenyl isocyanate (O. Rohr et al. DE 2,436,108) and 2-methoxy-5-(trifluoromethyl)phenyl isocyanate (K. Inukai et al. JP 42,025,067; *IBID Kogyo Kagaku Zasshi* **70**, *1967,* 491) have previously been described.

Thin-layer chromatography (TLC) was performed using Whatman^{®} pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science^{®} silica gel.

Melting points (mp) were determined using a Thomas-Hoover melting point apparatus or a Mettler FP66 automated melting point apparatus and are uncorrected. Fourier transform infrared sprectra were obtained using a Mattson 4020 Galaxy Series spectrophotometer. Proton (¹H) nuclear magnetic resonance (NMR) spectra were measured with a General Electric GN-Omega 300 (300 MHz) spectrometer with either Me₄Si (δ 0.00) or residual protonated solvent (CHCl₃ δ 7.26; MeOH δ 3:30; DMSO δ 2.49) as standard. Carbon (¹³C) NMR spectra were measured with a General Electric GN-Omega 300 (75 MHz) spectrometer with solvent (CDCl₃ δ 77.0; MeOD-d₃; δ 49.0; DMSO-d₆ δ 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250°C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer.. Chemical ionization mass spectra (CI-MS) were obtained using a Hewlett Packard MS-Engine (5989A) with methane or ammonia as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (~1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV). Elemental analyses were conducted by Robertson Microlit Labs, Madison NJ.

All compounds displayed NMR spectra, LRMS and either elemental analysis or HRMS consistant with assigned structures.

### List of Abbreviations and Acronyms:

- AcOH: acetic acid
- anh: anhydrous
- atm: atmosphere(s)
- BOC: *tert*-butoxycarbonyl
- CDI: 1,1'-carbonyl diimidazole
- conc: concentrated
- dec: decomposition
- DMAC: *N,N*-dimethylacetamide
- DMPU: 1,3-dimethyl-3,4,5,6-tetrahydro-2(1H)-pyrimidinone
- DMF: *N,N*-dimethylformamide
- DMSO: dimethylsulfoxide
- DPPA: diphenylphosphoryl azide
- EDCI: 1-(3-dimethylaminopropyl)-3-ethytcarbodiimide
- EtOAc: ethyl acetate
- EtOH: ethanol (100%)
- Et₂O: diethyl ether
- Et₃N: triethylamine
- HOBT: 1-hydroxybenzotriazole
- *m*-CPBA: 3-chloroperoxybenzoic acid
- MeOH: methanol
- pet. ether: petroleum ether (boiling range 30-60 °C)
- THF: tetrahydrofuran
- TFA: trifluoroacetic acid
- Tf: trifluoromethanesulfonyl

### A. General Methods for Synthesis of Substituted Anilines

### A1. General Method for Substituted Aniline Formation via Hydrogenation of a Nitroarene

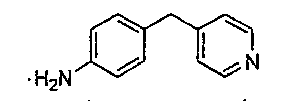

**4-(4-Pyridinylmethyl)aniline:** To a solution of 4-(4-nitrobenzyl)pyridine (7.0 g, 32.68 mmol) in EtOH (200 mL) was added 10% Pd/C (0.7 g) and the resulting slurry was shaken under a H₂ atmosphere (50 psi) using a Parr shaker. After 1 h, TLC and ¹H-NMR of an aliquot indicated complete reaction. The mixture was filtered through a short pad of Celite^{®}. The filtrate was concentrated in *vacuo* to afford a white solid (5.4 g, 90%): ¹H-NMR (DMSO-d₆) δ 3.74 (s, 2H), 4.91 (br s, 2H), 6.48 (d, *J*=8.46 Hz, 2H), 6.86 (d, *J*=8.09 Hz, 2H), 7.16 (d, *J*=5.88 Hz, 2H), 8.40 (d, *J*=5.88 Hz, 2H); EI-MS *m*/*z* 184 (M⁺). This material was used in urea formation reactions without further purification.

### A2. General Method for Substituted Aniline Formation via Dissolving Metal Reduction of a Nitroarene

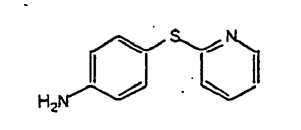

**4-(2-Pyridinylthio)aniline:** To a solution of 4-(2-pyridinylthio)-1-nitrobenzene (Menai ST 3355A; 0.220 g, 0.95 mmol) and H₂O (0.5 mL) in AcOH (5 mL) was added iron powder (0.317 g, 5.68 mmol) and the resulting slurry stirred for 16 h at room temp. The reaction mixture was diluted with EtOAc (75 mL) and H₂O (50 mL), basified to pH 10 by adding solid K₂CO₃ in portions *(**Caution**:* foaming). The organic layer was washed with a saturated NaCl solution, dried (MgSO₄), concentrated *in vacuo*. The residual solid was purified by MPLC (30% EtOAc/70% hexane) to give the desired product as a thick oil (0.135 g, 70%): TLC (30% EtOAc/70% hexanes) R*_{f}* 0.20.

### A3a. General Method for Substituted Aniline Formation via Nitroarene Formation Through Nucleophilic Aromatic Substitution, Followed by Reduction

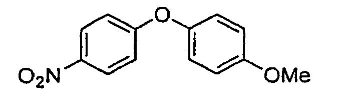

**Step 1. 1-Methoxy-4-(4-nitrophenoxy)benzene:** To a suspension of NaH (95%, 1.50 g, 59 mmol) in DMF (100 mL) at room temp. was added dropwise a solution of 4-methoxyphenol (7.39 g, 59 mmol) in DMF (50 mL). The reaction was stirred 1 h, then a solution of 1-fluoro-4-nitrobenzene (7.0 g, 49 mmol) in DMF (50 mL) was added dropwise to form a dark green solution. The reaction was heated at 95 °C overnight, then cooled to room temp., quenched with H₂O, and concentrated *in vacuo.* The residue was partitioned between EtOAc (200 mL) and H₂O (200 mL). The organic layer was sequentially washed with H₂O (2 x 200 mL), a saturated NaHCO₃ solution (200 mL), and a saturated NaCl solution (200 mL), dried (Na₂SO₄), and concentrated *in vacuo.* The residue was triturated (Et₂O/hexane) to afford 1-methoxy-4-(4-nitrophenoxy)benzene (12.2 g, 100%): ¹H-NMR (CDCl₃) δ 3.83 (s, 3H), 6.93-7.04 (m, 6H), 8.18 (d, *J*=9.2 Hz, 2H); EI-MS *m*/*z* 245 (M⁺).

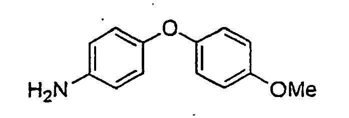

**Step 2. 4-(4-Methoxyphenoxy)aniline:** To a solution of 1-methoxy-4-(4-nitrophenoxy)benzene (12.0 g, 49 mmol) in EtOAc (250 mL) was added 5% Pt/C (1.5 g) and the resulting slurry was shaken under a H₂ atmosphere (50 psi) for 18 h. The reaction mixture was filtered through a pad of Celite^{®} with the aid of EtOAc and concentrated *in vacuo* to give an oil which slowly solidified (10.6 g, 100%): ¹H-NMR (CDCl₃) δ 3.54 (br s, 2H), 3.78 (s, 3H), 6.65 (d, *J*=8.8 Hz, 2H), 6.79-6.92 (m, 6H); EI-MS *m*/*z* 215 (M⁺).

### A3b. General Method for Substituted Aniline Formation via Nitroarene Formation Through Nucleophilic Aromatic Substitution, Followed by Reduction

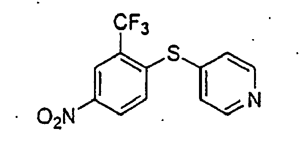

**Step 1. 3-(Trifluoromethyl)-4-(4-pyridinylthio)nitrosenzene:** A solution of 4-mercaptopyridine (2.8 g, 24 mmoles), 2-fluoro-5-nitrobenzotrifluoride (5 g, 23.5 mmoles), and potassium carbonate (6.1 g, 44.3 mmoles) in anhydrous DMF (80 mL) was stirred at room temperature and under argon overnight. TLC showed complete reaction. The mixture was diluted with Et₂O (100 mL) and water (100 mL) and the aqueous layer was back-extracted with Et₂O (2 x 100 mL). The organic layers were washed with a saturated NaCl solution (100 mL), dried (MgSO₄), and concentrated under reduced pressure. The solid residue was triturated with Et₂O to afford the desired product as a tan solid (3.8 g, 54%): TLC (30% EtOAc/70% hexane) R*_{f}* 0.06; ¹H-NMR (DMSO-d₆) δ 7.33 (dd, *J*=1.2, 4.2 Hz, 2H), 7.78 (d, *J*=8.7 Hz, 1H), 8.46 (dd, *J*=2.4, 8.7Hz, 1H), 8.54-8.56 (m, 3H).

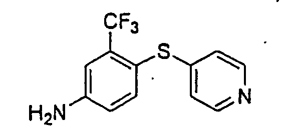

**Step 2. 3-(Trifluoromethyl)-4-(4-pyridinylthio)aniline:** A slurry of 3-trifluoromethyl-4-(4-pyridinylthio)nitrobenzene (3.8 g, 12.7 mmol), iron powder (4.0 g, 71.6 mmol), acetic acid (100 mL), and water (1 mL) were stirred at room temp. for 4 h. The mixture was diluted with Et₂O (100 mL) and water (100 mL). The aqueous phase was adjusted to pH 4 with a 4 N NaOH solution. The combined organic layers were washed with a saturated NaCl solution (100 mL), dried (MgSO₄), and concentrated under reduced pressure. The residue was filtered through a pad of silica (gradient from 50% EtOAc/50% hexane to 60% EtOAc/40% hexane) to afford the desired product (3.3 g): TLC (50% EtOAc/50% hexane) R*_{f}* 0.10; ¹H-NMR (DMSO-d₆) δ 6.21 (s, 2H), 6.84-6.87 (m, 3H), 7.10 (d, *J*=2.4 Hz, 1H), 7.39 (d, *J*=8.4 Hz, 1H), 8.29 (d, *J*=6.3 Hz, 2H).

### A3c. General Method for Substituted Aniline Formation via Nitroarene Formation Through Nucleophilic Aromatic Substitution, Followed by Reduction

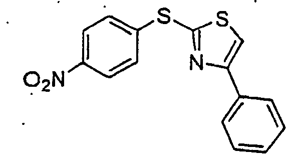

**Step 1. 4-(2-(4-Phenyl)thiazolyl)thio-1-nitrobenzene:** A solution of 2-mercapto-4-phenylthiazole (4.0 g, 20.7 mmoles) in DMF (40 mL) was treated with 1-fluoro-4-nitrobenzene (2.3 mL, 21.7 mmoles) followed by K₂CO₃ (3.18 g, 23 mmol), and the mixture was heated at approximately 65 °C overnight. The reaction mixture was then diluted with EtOAc (100 mL), sequentially washed with water (100 mL) and a saturated NaCl solution (100 mL), dried (MgSO₄) and concentrated under reduced pressure. The solid residue was triturated with a Et₂O/hexane solution to afford the desired product (6.1 g): TLC (25% EtOAc/75% hexane) R*_{f}* 0.49; ¹H-NMR (CDCl₃) δ 7.35-7.47 (m, 3H), 7.58-7.63 (m, 3H), 7.90 (d, *J*=6.9 Hz, 2H), 8.19 (d, *J*=9.0 Hz, 2H).

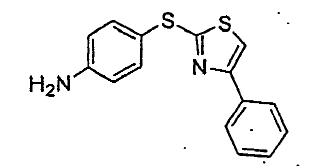

**Step 2. 4-(2-(4-Phenyl)thiazolyl)thioaniline:** 4-(2-(4-Phenyl)thiazolyl)thio-1-nitrobenzene was reduced in a manner analagous to that used in the preparation of 3-(trifluoromethyl)-4-(4-pyridinylthio)aniline: TLC (25% EtOAc/75% hexane) R*_{f}* 0.18; ¹H-NMR (CDCl₃) δ 3.89 (br s, 2H), 6.72-6.77 (m, 2H), 7.26-7.53 (m, 6H), 7.85-7.89 (m, 2H).

### A3d. General Method for Substituted Aniline Formation via Nitroarene Formation Through Nucleophilic Aromatic Substitution, Followed by Reduction

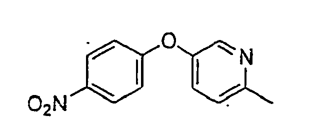

**Step 1. 4-(6-Methyl-3-pyridinylory)-1-nitrobenzene:** To a solution of 5-hydroxy-2-methylpyridine (5.0 g, 45.8 mmol) and 1-fluoro-4-nitrobenzene (6.5 g, 45.8 mmol) in anh DMF (50 mL) was added K₂CO₃ (13.0 g, 91.6 mmol) in one portion. The mixture was heated at the reflux temp. with stirring for 18 h and then allowed to cool to room temp. The resulting mixture was poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* to afford the desired product (8.7 g, 83%). This material was carried to the next step without further purification.

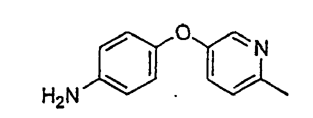

**Step 2. 4-(6-Methyl-3-pyridinyloxy)aniline:** A solution of 4-(6-methyl-3-pyridinyloxy)-1-nitrobenzene (4.0 g, 17.3 mmol) in EtOAc (150 mL) was added to 10% Pd/C (0.500 g, 0.47 mmol) and the resulting mixture was placed under a H₂ atmosphere (balloon) and was allowed to stir for 18 h at room temp. The mixture was then filtered through a pad of Celite^{®} and concentrated *in vacuo* to afford the desired product as a tan solid (3.2 g, 92%): EI-MS *m*/*z* 200 (M⁺).

### A3e. General Method for Substituted Aniline Formation via Nitroarene Formation Through Nucleophilic Aromatic Substitution, Followed by Reduction

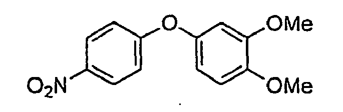

**Step 1. 4-(3,4-Dimethoxyphenoxy)-1-nitrobenzene:** To a solution of 3,4-dimethoxyphenol (1.0 g, 6.4 mmol) and 1-fluoro-4-nitrobenzene (700 µL, 6.4 mmol) in anh DMF (20 mL) was added K₂CO₃ (1.8 g, 12.9 mmol) in one portion. The mixture was heated at the reflux temp with stirring for 18 h and then allowed to cool to room temp. The mixture was then poured into water (100 mL) and extracted with EtOAc (3 x 100 mL). The combined organics were sequentially washed with water (3 x 50 mL) and a saturated NaCl solution (2 x 50 mL), dried (Na₂SO₄), and concentrated *in vacuo* to afford the desired product (0.8 g, 54%). The crude product was carried to the next step without further purification.

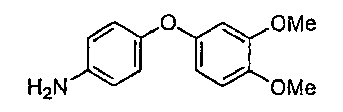

**Step 2. 4-(3,4-Dimethoryphenoxy)aniline:** A solution of 4-(3,4-dimethoxy-phenoxy)-1-nitrobenzene (0.8 g, 3.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a white solid (0.6 g, 75%): EI-MS *m*/*z* 245 (M⁺).

### A3f. General Method for Substituted Aniline Formation via Nitroarene Formation Through Nucleophilic Aromatic Substitution, Followed by Reduction

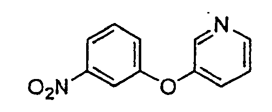

**Step 1. 3-(3-Pyridinyloxy)-1-nitrobenzene:** To a solution of 3-hydroxypyridine (2.8 g, 29.0 mmol), 1-bromo-3-nitrobenzene (5.9 g, 29:0 mmol) and copper(I) bromide (5.0 g, 34.8 mmol) in anh DMF (50 mL) was added K₂CO₃ (8.0 g, 58.1 mmol) in one portion. The resulting mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (2.0 g, 32 %). This material was used in the next step without further purification.

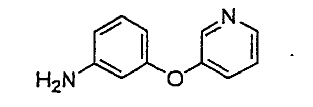

**Step 2. 3-(3-Pyridinyloay)aniline:** A solution of 3-(3-pyridinyloxy)-1-nitrobenzene (2.0 g, 9.2 mmol) in EtOAc (100 mL) was added to 10% Pd/C (0.200 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a red oil (1.6 g, 94%): EI-MS *m*/*z* 186 (M⁺).

### A3g. General Method for Substituted Aniline Formation via Nitroarene Formation Through Nucleophilic Aromatic Substitution, Followed by Reduction

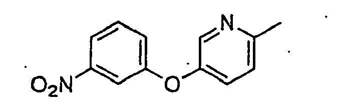

**Step 1. 3-(5-Methyl-3-pyridinyloxy)-1-nitrobenzene:** To a solution of 3-hydroxy-5-methylpyridine (5.0 g, 45.8 mmol), 1-bromo-3-nitrobenzene (12.0 g, 59.6 mmol) and copper(I) iodide (10.0 g, 73.3 mmol) in anh DMF (50 mL) was added K₂CO₃ (13.0 g, 91.6 mmol) in one portion. The mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (1.2 g, 13%).

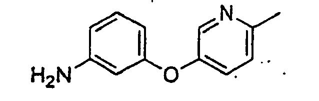

**Step 2. 3-(5-Methyl-3-pyridinyloxy)-1-nitrobenzene:** A solution of 3-(5-methyl-3-pyridinyloxy)-1-nitrobenzene (1.2 g, 5.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a red oil (0.9 g, 86%): CI-MS m/z 201 ((M+H)⁺).

### A3h. General Method for Substituted Aniline Formation via Nitroarene Formation Through Nucleophilic Aromatic Substitution, Followed by Reduction

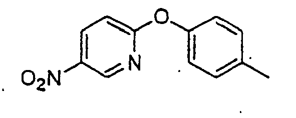

**Step 1. 5-Nitro-2-(4-methylphenory)pyridine:** To a solution of 2-chloro-5-nitropyridine (6.34 g, 40 mmol) in DMF (200 mL) were added of 4-methylphenol (5.4 g, 50 mmol, 1.25 equiv) and K₂CO₃ (8.28 g, 60 mmol, 1.5 equiv). The mixture was stirred overnight at room temp. The resulting mixture was treated with water (600 mL) to generate a precipitate. This mixture was stirred for 1 h, and the solids were separated and sequentially washed with a 1 N NaOH solution (25 mL), water (25 mL) and pet ether (25 mL) to give the desired product (7.05 g, 76%): mp 80-82 °C; TLC (30% EtOAc/70% pet ether) R*_{f}* 0.79; ¹H-NMR (DMSO-d₆) δ 2.31 (s, 3H), 7.08 (d, *J*=8.46 Hz, 2H), 7.19 (d, *J*=9.20 Hz, 1H), 7.24 (d, *J*=8.09 Hz, 2H), 8.58 (dd, *J*=2.94, 8.82 Hz, 1H), 8.99 (d, *J*=2.95 Hz, 1H); FAB-MS *m*/*z* (rel abundance) 231 ((M+H)⁺), 100%).

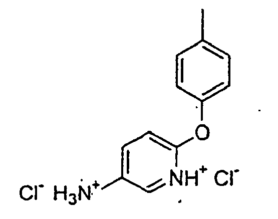

**Step 2. 5-Amino-2-(4-methylphenoxy)pyridine Dihydrochloride:** A solution 5-nitro-2-(4-methylphenoxy)pyridine (6.94 g, 30 mmol, 1 eq) and EtOH (10 mL) in EtOAc (190 mL) was purged with argon then treated with 10% Pd/C (0.60 g). The reaction mixture was then placed under a H₂ atmosphere and was vigorously stirred for 2.5 h. The reaction mixture was filtered through a pad of Celite^{®}. A solution of HCl in Et₂O was added to the filtrate was added dropwise. The resulting precipitate was separated and washed with EtOAc to give the desired product (7.56 g, 92%): mp 208-210 °C (dec); TLC (50% - EtOAc/50% pet ether) R*_{f}* 0.42; ¹H-NMR (DMSO-d₆) δ 2.25 (s, 3H), 6.98 (d, *J*=8.45 Hz, 2H), 7.04 (d, *J*=8.82 Hz, 1H), 7.19 (d, *J*=8.09 Hz, 2H), 8.46 (dd, *J*=2.57, 8.46 Hz, 1H), 8.63 (d, *J*=2.57 Hz, 1H); EI-MS *m*/*z* (rel abundance) (M⁺, 100%).

### A3i. General Method for Substituted Aniline Formation via Nitroarene Formation Through Nucleophilic Aromatic Substitution, Followed by Reduction

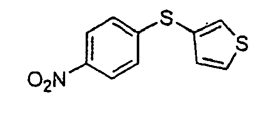

**Step 1. 4-(3-Thienylthio)-1-nitrobenzene:** To a solution of 4-nitrothiophenol (80%pure; 1.2 g, 6.1 mmol), 3-bromothiophene (1.0 g, 6.1 mmol) and copper(II) oxide (0.5 g, 3.7 mmol) in anhydrous DMF (20 mL) was added KOH (0.3 g, 6.1 mmol), and the resulting mixture was heated at 130 °C with stirring for 42 h and then allowed to cool to room temp. The reaction mixture was then poured into a mixture of ice and a 6N HCl solution (200 mL) and the resulting aqueous mixture was extracted with EtOAc (3 x 100 mL). The combined organic layers were sequentially washed with a 1M NaOH solution (2 x 100 mL) and a saturated NaCl solution (2 x 100 mL), dried (MgSO₄), and concentrated *in vacuo*. The residual oil was purified by MPLC (silica gel; gradient from 10% EtOAc/90% hexane to 5% EtOAc/95% hexane) to afford of the desired product (0.5 g, 34%). GC-MS *m*/*z* 237 (M⁺).

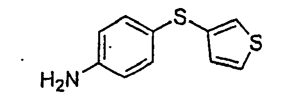

**Step 2. 4-(3-Thienylthio)aniline:** 4-(3-Thienylthio)-1-nitrobenzene was reduced to the aniline in a manner analogous to that described in Method A1.

### A3j. General Method for Substituted Aniline Formation via Nitroarene Formation Through Nucleophilic Aromatic Substitution, Followed by Reduction

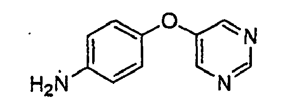

**4-(5-Pyrimininyloxy)aniline:** 4-Aminophenol (1.0 g, 9.2 mmol) was dissolved in DMF (20 mL) then 5-bromopyrimidine (1.46 g, 9.2 mmol) and K₂CO₃ (1.9 g, 13.7 mmol) were added. The mixture was heated to 100 °C for 18 h and at 130 °C for 48 h at which GC-MS analysis indicated some remaining starting material. The reaction mixture was cooled to room temp. and diluted with water (50 mL). The resulting solution was extracted with EtOAc (100 mL). The organic layer was washed with a saturated NaCl solution (2 x 5.0 mL), dried (MgSO₄), and concentrated *in vacuo.* The residual solids were purified by MPLC (50% EtOAc/50% hexanes) to give the desired amine (0.650 g, 38%).

### A3k. General Method for Substituted Aniline Formation via Nitroarene Formation Through Nucleophilic Aromatic Substitution, Followed by Reduction

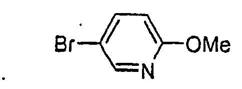

**Step 1. 5-Bromo-2-methoxypyridine:** A mixture of 2,5-dibromopyridine (5.5 g, 23.2 mmol) and NaOMe (3.76g, 69.6 mmol) in MeOH (60 mL) was heated at 70 °C in a sealed reaction vessel for 42 h, then allowed to cool to room temp. The reaction mixture was treated with water (50 mL) and extracted with EtOAc (2 x 100 mL). The combined organic layers were dried (Na₂SO₄) and concentrated under reduced pressure to give a pale yellow, volatile oil (4.1g, 95% yield): TLC (10% EtOAc / 90% hexane) R*_{f}* 0.57.

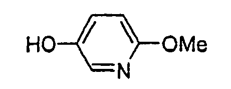

**Step 2. 5-Hydroxy-2-methoxypyridine:** To a stirred solution of 5-bromo-2-methoxypyridine (8.9 g, 47.9 mmol) in THF (175 mL) at -78 °C was added an n-butyllithium solution (2.5 M in hexane; 28.7 mL, 71.8 mmol) dropwise and the resulting mixture was allowed to stir at -78 °C for 45 min. Trimethyl borate (7.06 mL, 62.2 mmol) was added via syringe and the resulting mixture was stirred for an additional 2 h. The bright orange reaction mixture was warmed to 0 °C and was treated with a mixture of a 3 N NaOH solution (25 mL, 71.77 mmol) and a hydrogen peroxide solution (30%; approx. 50 mL). The resulting yellow and slightly turbid reaction mixture was warmed to room temp. for 30 min and then heated to the reflux temp. for 1 h. The reaction mixture was then allowed to cool to room temp. The aqueous layer was neutralized with a 1N HCl solution then extracted with Et₂O (2 x 100 mL). The combined organic layers were dried (Na₂SO₄) and concentrated under reduced pressure to give a viscous yellow oil (3.5g, 60%).

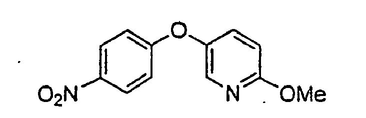

**Step 3. 4-(5-(2-Methoxy)pyridyl)oxy-1-nitrobenzene:** To a stirred slurry of NaH (97%, 1.0 g, 42 mmol) in anh DMF (100 mL) was added a solution of 5-hydroxy-2-methoxypyridine (3.5g, 28 mmol) in DMF (100 mL). The resulting mixture was allowed to stir at room temp. for 1 h, 4-fluoronitrobenzene (3 mL, 28 mmol) was added via syringe. The reaction mixture was heated to 95 °C overnight, then treated with water (25 mL) and extracted with EtOAc (2 x 75 mL). The organic layer was dried (MgSO₄) and concentrated under reduced pressure. The residual brown oil was crystalized EtOAc/hexane) to afford yellow crystals (5.23 g, 75%).

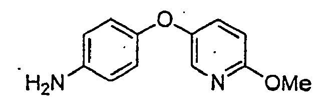

**Step 4. 4-(5-(2-Methoxy)pyridyl)oryaniline:** 4-(5-(2-Methoxy)pyridyl)oxy-1-nitrobenzene was reduced to the aniline in a manner analogous to that described in Method A3d, Step2.

### A4a. General Method for Substituted Aniline Synthesis via Nucleophilic Aromatic Substitution using a Halopyridine

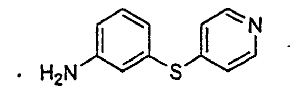

3-(4-Pyridinylthio)aniline: To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed with a saturated NaCl solution (100 mL), dried (MgSO₄), and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R*_{f}* 0.29; ¹H-NMR (DMSO-d₆) δ 5.41 (s, 2H), 6.64-6.74 (m, 3H), 7.01 (d, J=4.8, 2H), 7.14 (t, J=7.8 Hz, 1H), 8.32 (d, J=4.8, 2H).

### A4b. General Method for Substituted Aniline Synthesis via Nucleophilic Aromatic Substitution using a Halopyridine

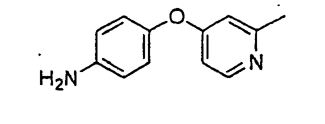

**4-(2-Methyl-4-pyridinyloxy)aniline:** To a solution of 4-aminophenol (3.6 g, 32.8 mmol) and 4-chloropicoline (5.0 g, 39.3 mmol) in anh DMPU (50 mL) was added potassium *tert-*butoxide (7.4 g, 65.6 mmol) in one portion. The reaction mixture was heated at 100 °C with stirring for 18 h, then was allowed to cool to room temp. The resulting mixture was poured into water (200 mL) and extracted with EtOAc (3 x 150 mL). The combined extracts were sequentially washed with water (3 x 100 mL) and a saturated NaCl solution (2 x 100 mL), dried (Na₂SO₄), and concentrated *in vacuo.* The resulting oil was purified by flash chromatography (50 % EtOAc/50% hexane) to afford the desired product as a yellow oil (0.7 g, 9%): CI-MS *m*/*z* 201 ((M+H)⁺).

### A4c. General Method for Substituted Aniline Synthesis via Nucleophilic Aromatic Substitution using a Halopyridine

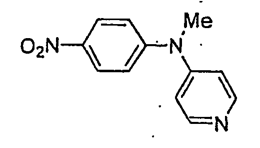

**Step 1. Methyl(4-nitrophenyl)-4-pyridylamine:** To a suspension of *N*-methyl-4-nitroaniline (2.0 g, 13.2 mmol) and K₂CO₃ (7.2 g, 52.2 mmol) in DMPU (30mL) was added 4-chloropyridine hydrochloride (2.36 g, 15.77 mmol). The reaction mixture was heated at 90 °C for 20 h, then cooled to room temperature. The resulting mixture was diluted with water (100 mL) and extracted with EtOAc (100 mL). The organic layer was washed with water (100 mL), dried (Na₂SO₄) and concentrated under reduced pressure. The residue was purified by column chromatography (silica gel, gradient from 80% EtOAc /26% hexanes to 100% EtOAc) to afford methyl(4-nitrophenyl)-4-pyridylamine (0.42 g) -

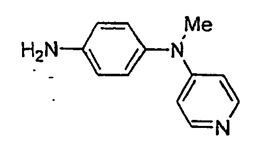

**Step 2. Methyl(4-aminophenyl)-4-pyridylamine:** Methyl(4-nitrophenyl)-4-pyridylamine was reduced in a manner analogous to that described in Method A1.

### A5. General Method of Substituted Aniline Synthesis via Phenol Alkylation Followed by Reduction of a Nitroarene

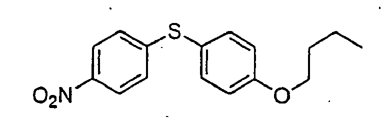

**Step 1. 4-(4-Butoxyphenyl)thio-1-nitrobenzene:** To a solution of 4-(4-nitrophenylthio)phenol (1.50 g, 6.07 mmol) in anh DMF (75 ml) at 0 °C was added NaH (60% in mineral oil, 0.267 g, 6.67 mmol). The brown suspension was stirred at 0 °C until gas evolution stopped (15 min), then a solution of iodobutane (1.12 g, .690 ml, 6.07 mmol) in anh DMF (20 mL) was added dropwise over 15 min at 0 °C. The reaction was stirred at room temp. for 18 h at which time TLC indicated the presence of unreacted phenol, and additional iodobutane (56 mg, 0.035 mL, 0.303 mmol, 0.05 equiv) and NaH (13 mg, 0.334 mmol) were added. The reaction was stirred an additional 6 h at room temp., then was quenched by the addition of water (400 mL). The resulting mixture was extracted with Et₂O (2 x 500 mL). The combined organics were washed with water (2 x 400 mL), dried (MgSO₄), and concentrated under reduced pressure to give a clear yellow oil, which was purified by silica gel chromatography (gradient from 20% EtOAc/80% hexane to 50% EtOAc/50% hexane) to give the product as a yellow solid (1.24 g, 67%): TLC (20% EtOAc/80% hexane) R*_{f}* 0.75; ¹H-NMR (DMSO-d₆) δ 0.92 (t, *J*= 7.5 Hz, 3H), 1.42 (app hex, *J*=7.5 Hz, 2H), 1.70 (m, 2H), 4.01. (t, *J=* 6.6 Hz, 2H), 7.08 (d, *J*=8.7 Hz, 2H), 7.17 (d, *J*=9 Hz, 2H), 7.51 (d, *J*= 8.7 Hz, 2H), 8.09 (d, *J*= 9 Hz, 2H).

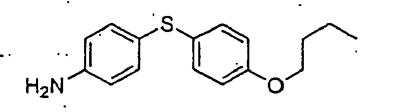

**Step 2. 4-(4-Butoxyphenyl)thioaniline:** 4-(4-Butoxyphenyl)thio-1-nitrobenzene was reduced to the aniline in a manner analagous to that used in the preparation of 3-(trifluoromethyl)-4-(4-pyridinylthio)aniline (Method A3b, Step 2): TLC (33% EtOAc/77% hexane) R*_{f}* 0.38.

### A6. General Method for Synthesis of Substituted Anilines by the Acylation of Diaminoarenes

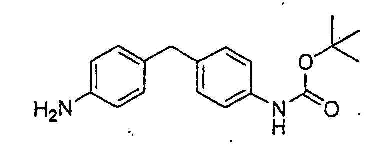

**4-(4-*tert*-Butoxycarbamoylbenzyl)aniline:** To a solution of 4,4'-methylenedianiline (3.00 g, 15.1 mmol) in anh THF (50 mL) at room temp was added a solution of di-tert-butyl dicarbonate (3.30 g, 15.1 mmol) in anh THF (10 mL). The reaction mixture was heated at the reflux temp. for 3 h, at which time TLC indicated the presence of unreacted methylenedianiline. Additional di-*tert*-butyl dicarbonate (0.664 g, 3.03 mmol, 0.02 equiv) was added and the reaction stirred at the reflux temp. for 16 h. The resulting mixture was diluted with Et₂O (200 mL), sequentially washed with a saturated NaHCO₃ solution (100 ml), water (100 mL) and a saturated NaCl solution (50 mL), dried (MgSO₄), and concentrated under reduced pressure. The resulting white solid was purified by silica gel chromatography (gradient from 33% EtOAc/67% hexane to 50% EtOAc/50% hexane) to afford the desired product as a white solid (2.09 g, 46%): TLC (50% EtOAc/50% hexane) R*_{f}* 0.45; ¹H-NMR (DMSO-d₆) δ 1.43 (s, 9H), 3.63 (s, 2H), 4.85 (br s, 2H), 6.44 (d, *J*=8.4 Hz, 2H), 6.80 (d, *J*=8.1 Hz, 2H), 7.00 (d, *J*=8.4 Hz, 2H), 7.28 (d, *J*=8.1 Hz, 2H), 9.18 (br s, 1H); FAB-MS *m*/*z* 298 (M⁺).

### A7. General Method for the Synthesis of Aryl Amines via Electrophilic Nitration Followed by Reduction

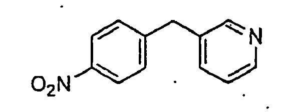

**Step 1. 3-(4-Nitrobenzyl)pyridine:** A solution of 3-benzylpyridine (4.0 g, 23.6 mmol) and 70% nitric acid (30 mL) was heated overnight at 50 °C. The resulting mixture was allowed to cool to room temp. then poured into ice water (350 mL). The aqueous mixture then made basic with a 1N NaOH solution, then extracted with Et₂O (4 x 100 mL). The combined extracts were sequentially washed with water (3 x 100 mL) and a saturated NaCl solution (2 x 100 mL), dried (Na₂SO₄), and concentrated *in vacuo*. The residual oil was purified by MPLC (silica gel; 50 % EtOAc/50% hexane) then recrystallization (EtOAc/hexane) to afford the desired product (1.0 g, 22%): GC-MS *m*/*z* 214 (M⁺).

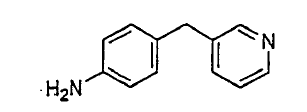

Step 2. 3-(4-Pyridinyl)methylaniline: 3-(4-Nitrobenzyl)pyridine was reduced to the aniline in a manner analogous to that described in Method A1.

### A8. General Method for Synthesis of Aryl Amines via Substitution with Nitrobenzyl Halides Followed by Reduction

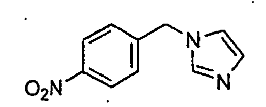

Step 1. 4-(1-Imidazolylmethyl)-1-nitrobenzene: To a solution of imidazole (0.5 g, 7.3 mmol) and 4-nitrobenzyl bromide (1.6 g, 7.3 mmol) in anh acetonitrile (30 mL) was added K₂CO₃ (1.0 g, 7.3 mmol). The resulting mixture was stirred at room temp. for 18 h and then poured into water (200 mL) and the resulting aqueous solution was extracted with EtOAc (3 x 50 mL). The combined organic layers were sequentially washed with water (3 x 50 mL) and a saturated NaCl solution (2 x 50 mL), dried (MgSO₄), and concentrated *in vacuo.* The residual oil was purified by MPLC (silica gel; 25% EtOAc/75% hexane) to afford the desired product (1.0 g, 91%): EI-MS *m*/*z* 203 (M⁺).

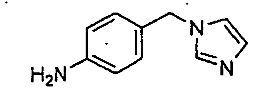

Step 2. 4-(1-Imidazolylmethyl)aniline: 4-(1-Imidazolylmethyl)-1-nitrobenzene was reduced to the aniline in a manner analogous to that described in Method A2.

### A9. Formation of Substituted Hydroxymethylanilines by Oxidation of Nitrobenzyl Compounds Followed by Reduction

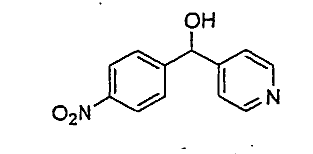

**Step 1. 4-(1-Hydroxy-1-(4-pyridyl)methyl-1-nitrobenzene:** To a stirred solution of 3-(4-nitrobenzyl)pyridine (6.0 g, 28 mmol) in CH₂Cl₂ (90 mL) was added *m*-CPBA (5.80 g, 33.6 mmol) at 10 °C, and the mixture was stirred at room temp. overnight. The reaction mixture was successively washed with a 10% NaHSO₃ solution (50 mL), a saturated K₂CO₃ solution (50 mL) and a saturated NaCl solution (50 mL), dried (MgSO₄) and concentrated under reduced pressure. The resulting yellow solid (2.68 g) was dissolved in anh acetic anhydride (30 mL) and heated at the reflux temperature overnight. The mixture was concentrated under reduced pressure. The residue was dissolved in MeOH (25 mL) and treated with a 20% aqueous NH₃ solution (30 mL). The mixture was stirred at room temp. for 1 h, then was concentrated under reduced pressure. The residue was poured into a mixture of water (50 mL) and CH₂Cl₂ (50 mL). The organic layer was dried (MgSO₄), concentrated under reduced pressure, and purified by column chromatography (80% EtOAc/20% hexane) to afford the desired product as a white solid. (0.53 g, 8%): mp 110-118 °C; TLC (80% EtOAc/20% hexane) R*_{f}* 0.12; FAB-MS *m*/*z* 367 ((M+H)⁺, 100%).

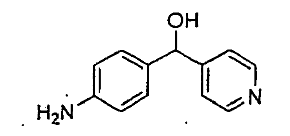

**Step 2. 4-(1-Hydroxy-1-(4-pyridyl)methylaniline:** 4-(1-Hydroxy-1-(4-pyridyl)-methyl-1-nitrobenzene was reduced to the aniline in a manner analogous to that described in Method A3d, Step2.

### A10. Formation of 2-(N-methylcarbamoyl)pyridines via the Menisci reaction

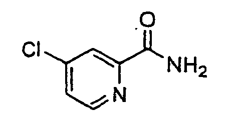

**Step 1. 2-(*N*-methylcarbamoyl)-4-chloropyridine. (Caution:** this is a highly hazardous, potentially explosive reaction.) To a solution of 4-chloropyridine (10.0 g) in *N-*methylformamide (250 mL) under argon at ambient temp was added conc. H₂SO₄ (3.55 mL) (exotherm). To this was added H₂O₂ (17 mL, 30% wt in H2O) followed by FeSO₄·7H₂O (0.55 g) to produce an exotherm. The reaction was stirred in the dark at ambient temp for 1h then was heated slowly over 4 h at 45 °C. When bubbling subsided,the reaction was heated at 60 °C for 16 h. The opaque brown solution was diluted with H2O (700 mL) followed by a 10% NaOH solution (250 mL). The aqueous mixture was extracted with EtOAc (3 x 500 mL) and the organic layers were washed separately with a saturated NaCl solution (3 x 150 mL. The combined organics were dried (MgSO₄) and filtered through a pad of silica gel eluting with EtOAc. The solvent was removed in vacuo and the brown residue was purified by silica gel chromatography (gradient from 50% EtOAc/ 50% hexane to 80% EtOAc / 20% hexane). The resulting yellow oil crystallized at 0 °C over 72 h to give 2-(*N*-methylcarbamoyl)-4-chloropyridine in yield (0.61 g, 5.3%): TLC (50% EtOAc/50% hexane) R*_{f}* 0.50; MS; ¹H NMR (CDCl₃): δd 8.44 (d, 1 H, J = 5.1 Hz, CHN), 8.21 (s, 1H, CHCCO), 7.96 (b s, 1H, NH), 7.43 (dd, 1H, J = 2.4, 5.4 Hz, ClCHCN), 3.04 (d, 3H, J = 5.1 Hz, methyl); CI-MS *m*/*z* 171 1 ((M+H)+).

### A11. General Method for the Synthesis of δ-Sulfonylphenyl Anilines

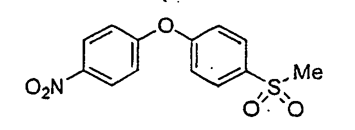

**Step 1. 4-(4-Methylsulfonylphenoxy)-1-nitrobenzene:** To a solution of 4-(4-methylthiophenoxy)-1-ntirobenzene (2 g, 7.66 mmol) in CH₂Cl₂ (75 mL) at 0 °C was slowly added *m*CPBA (57-86%, 4 g), and the reaction mixture was stirred at room temperature for 5 h. The reaction mixture was treated with a 1 N NaOH solution (25 mL). The organic layer was sequentially washed with a 1N NaOH solution (25 mL), water (25 mL) and a saturated NaCl solution (25 mL), dried (MgSO₄), and concentrated under reduced pressure to give 4-(4-methylsulfonylphenoxy)-1-nitrobenzene as a solid (2.1 g).

**Step 2. 4-(4-Methylsulfonylphenoxy)-1-aniline:** 4-(4-Methylsulfonylphenoxy)-1-nitrobenzene was reduced to the aniline in a manner anaologous to that described in Method A3d, step 2.

### A12. General Method for Synthesis of δ-Alkoxy-δ-carboxyphenyl Anilines

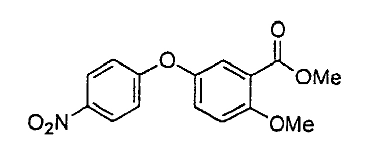

**Step 1. 4-(3-Methoxycarbonyl-4-methoxyphenoxy)-1-nitrobenzene:** To a solution of δ-(3-carboxy-4-hydroxyphenoxy)-1-nitrobenzene (prepared in a manner analogous to that described in Method A3a, step 1, 12 mmol) in acetone (50 mL) was added K₂CO₃ (5 g) and dimethyl sulfate (3.5 mL). The resulting mixture was heated at the reflux temperature overnight, then cooled to room temperature and filtered through a pad of Celite^{®}. The resulting solution was concentrated under reduced pressure, absorbed onto silica gel, and purified by column chromatography (50% EtOAc / 50% hexane) to give 4-(3-methoxycarbonyl-4-methoxyphenoxy)-1-nitrobenzene as a yellow powder (3 g): mp 115 - 118 °C.

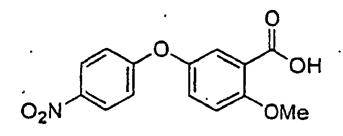

**Step 2. 4-(3-Carboxy-4-methoxyphenoxy)-1-nitrobenzene:** A mixture of 4-(3-methoxycarbonyl-4-methoxyphenoxy)-1-nitrobenzene (1.2 g), KOH (0.33 g),and water (5 mL) in MeOH (45 mL) was stirred at room temperature overnight and then heated at the reflux temperature for 4 h. The resulting mixture was cooled to room temperature and concentrated under reduced pressure. The residue was dissolved in water (50 mL), and the aqueous mixture was made acidic with a 1N HCl solution. The resulting mixture was extracted with EtOAc (50 mL). The organic layer was dried (MgSO₄) and concentrated under reduced pressure to give 4-(3-carboxy-4-methoxyphenoxy)-1-nitrobenzene (1.04 g).

### B. General Methods of Urea Formation

### B1. Reaction of a Heterocyclic Amine with an Aryl Isocyanate

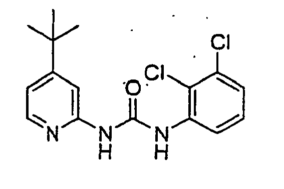

***N*-(4-*tert*-butylpyridyl)-*N'*-(2,3-dichlorophenyl) urea:** A solution of 2-amino-4-*tert-*butylpyridine (192 mg) and 2,3-dichlorophenyl isocyanate (240 mg) in anh. toluene (15 mL) was heated at 70 °C under argon for 24 h. The resulting mixture was diluted with EtOAc (200 mL) then washed with water (125 mL). The organic layer was dried (MgSO₄) and concentrated under reduced pressure to give a gum. Trituration of the gum with hexanes afforded *N*-(4-*tert*-butylpyridyl)-*N'*-(2,3-dichlorophenyl) urea as a white solid (394 mg, 91%): TLC (2:1 hexanes/ethyl acetate) R*_{f}* 0.40; FAB-MS *m*/*z* 338 ((M+H)⁺).

### B2a. Reaction of a Heterocyclic Amine with N,N'-Carbonyldiimidazole Followed by Reaction with a Substituted Aniline

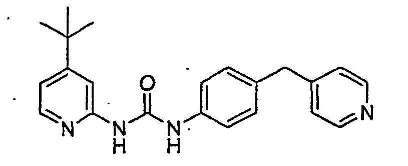

***N*-(4-*tert*-butylpyridyl)-*N'*-(4-(4-pyridinylmethyl)phenyl urea:** To a stirring solution of 4-*tert*-butyl-2-aminopyridine (192 mg) in anh. CH₂Cl₂ (15 mL) under argon at 0 °C was added CDI (207 mg). The resulting solution was allowed to warm to ambient temp over 2 h. To this mixture was added 4-(4-pyridylmethyl)aniline (prepared according to Method A1, 235 mg). The resulting solution was stirred at room temperature for 24 h, then was quenched with water (125 mL). The resulting mixture was extracted with EtOAc (200 mL). The organic layer was washed with water (100 mL), dried (MgSO₄) and concentrated under reduced pressure. The residue was purified by chromatography (SiO₂, EtOAc) to afford *N*-(4-*tert*-butylpyridyl)-*N'*-(4-(4-pyridinylmethyl)phenyl urea as a white solid (200 mg, 43%): TLC (EtOAc) R*_{f}* 0.47; FAB-MS *m*/*z* 361 ((M+H)⁺).

### B2b. Reaction of a Heterocyclic Amine with N,N'-Carbonyldiimidazole Followed by Reaction with a Substituted Aniline

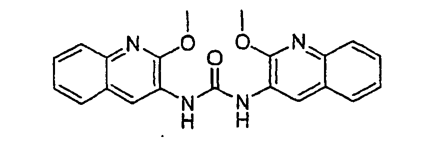

*N,N'*-(Bis(3-(2-methoxyquinolinyl)) urea): To a stirring solution of 3-amino-2-methoxyquinoline (138 mg) in anh. CH₂Cl₂ (15 mL) under argon at 0 °C was added CDI (128 mg). The resulting solution was warmed to ambient temp over 1 h. After 16 h 4-(2-*N*-Methylcarbamyl-4-pyridyloxy)aniline (175 mg) was added and the resulting yellow solution was stirred at room temperature under argon for 72 h. The solution was treated with water (125 mL) and the resulting mixture was extracted with EtOAc (2 x 150 mL). The combined organics were washed with a saturated NaCl solution (100 mL), dried (MgSO₄) and concentrated under reduced pressure. The residue was triturated with a 10% hexane/90% EtOAc solution. The resulting white crystals were washed with EtOAc. The resulting filtrate was purified by chromatography (SiO₂, 50% EtOAc/50% hexane) to give *N,N'*-(bis(3-(2-methoxyquinolinyl)) urea) (30 mg, 20% yield): TLC (50% EtOAc/50% hexane) R*_{f}* 0.45; HPLC ES-MS *m*/*z* 375 ((M+H)⁺).

### B2c. Reaction of a Heterocyclic Amine with N,N'-Carbonyldiimidazole Followed by Reaction with a Substituted Aniline

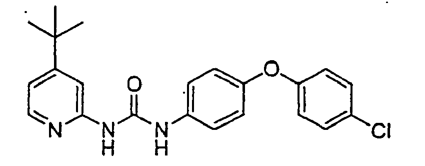

***N*-(4-*tert*-Butylpyridyl)-*N'*-(4-(4-chlorophenoxy)phenyl) urea:** A solution of 4-*tert-*butyl-2-aminopyridine (0.177 g, 1.18 mmol, 1 equiv.) in 1.2 mL of anh. CH₂Cl₂ (1.2 mL) was added to CDI (0.200 g, 1.24 mmol, 1.05 equiv) and the mixture was allowed to stir under argon at room temperature 1 d. To the resulting solution was added 4-(4-chlorophenoxy)aniline (0.259 g, 1.18 mmol, 1 equiv.) in one portion. The resulting mixture was stirred at room temperature for 1 d, then was treated with a 10% citric acid solution (2 mL) and allowed to stir for 1 h. The resulting organic layer was extracted with EtOAc (3 x 5 mL). The combined organic layers were dried (MgSO₄) and concentrated *in vacuo.* The resultant residue was treated with CH₂Cl₂ (10 mL) and a 1 N aqueous NaOH solution. This mixture was allowed to stir overnight. The resulting organic layer was extracted with CH₂Cl₂ (3 x 5 mL). The combined organic layers were (MgSO₄) and concentrated *in vacuo.* The resultant solids were suspended in diethyl ether (10 mL) and sonicated for 15 minutes. The resulting white solids were dried to give *N*-(4-*tert-*butylpyridyl)-*N'*-(4-(4-chlorophenoxy)phenyl) urea (42 mg, 9%): mp 193-199 °C.

### B3. Reaction of Substituted Aniline with N,N'-Carbonyldiimidazole Followed by Reaction with a Heterocyclic Amine

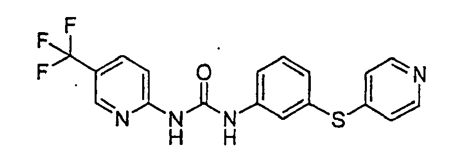

***N*-(2-(5-trifluoromethyl)pyridyloxy)-*N'*-(3-(4-pyridylthio)phenyl) urea:** A solution of 3-(4-pyridylthio)aniline (300 mg, 1.48 mmoles) in CH₂Cl₂ (12 mL) was treated with CDI (253 mg, 1.56 mmoles). The solution was stirred at room temperature and under argon for 2 h. The resulting mixture was treated.with 2-amino-5-(trifluoromethyl)pyridine (238 mg, 1.47 mmoles) and heated at 40 °C overnight. The reaction mixture was then diluted with EtOAc (25 mL), washed with water (10 mL) and a saturated NaCl solution m(25 mL), dried (MgSO₄), and concentrated under reduced pressure. The residue was purified by column chromatography (SiO₂; gradient from 70% EtOAc/30% CH₂Cl₂ to 100% EtOAc to give *N*-(2-(5-trifluoromethyl)pyridyloxy)-*N'*-(3-(4-pyridylthio)phenyl) urea (103 mg): TLC (50% EtOAc/50% CH₂Cl₂) R*_{f}* 0.33; ¹H-NMR (DMSO-d₆) δ 6.06 (d, *J*=6Hz, 2H), 7.25 (dt, *J*=1.2,7.8 Hz, 1H), 7.48 (t, *J*=8.1 Hz, 1H), 7.59-7.63 (m, 1H), 7.77 (d, *J*=8.7 Hz, 1H), 7.86 (t, *J*=1.8 Hz, 1H), 8.12 (dd, *J*=2.7,9.3 Hz, 1H), 8.37 (d, *J*=6.3 Hz, 2H), 8.67 (bs, 1H), 9.88 (s, 1H), 10.26 (s, 1 H); FAB-MS *m*/*z* 391 ((M+H)⁺).

### B4. Reaction of a Heterocyclic Amine with Phosgene, Followed by Reaction with a Substituted Aniline

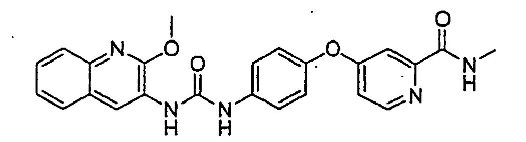

***N*-(3-(2-methoxyquinolinyl)-*N'*-(4-(4-(2-*N*-Methylcarbamyl-4-pyridyloxy)phenyl) urea:** To a stirring solution of phosgene (20% in toluene, 1.38 mL) in anh. CH₂Cl₂ (20 ml) at 0 °C under argon was added anh. pyridine (207 mg) followed by 3-amino-2-methoxyquinoline (456 mg). The resulting solution was warmed to ambient temperature over 1 h, then concentrated in vacuo at ambient temperature to give a white solid. The solid was dried under vacuum for 15 min then suspended in anh toluene (20 mL). To the resulting slurry was added 4-(4-(2-(methylcarbamoyl)pyridyloxy)aniline (prepared according to Method A2, 300 mg) and the reaction heated under argon at 80 °C for 20 h. The resulting mixture was diluted with water (200 mL), then treated with a saturated NaHCO₃ solution (10 mL) and extracted with EtOAc (2 x 300 mL). The combined organic layers were washed with a saturated NaCl solution (100 mL), dried (MgSO4) and concentrated under reduced pressure. The solid yellow residue was purified by chromatography (SiO₂, gradient from 50% EtOAc/50% hexane to 100% EtOAc), followed by recrystallization from diethyl ether and hexane to give *N*-(3-(2-methoxyquinolinyl)-*N*'-(4-(4-(2-*N*-Methylcarbamyl-4-pyridyloxy)phenyl) urea as a white solid (140 mg, 25%): TLC (EtOAc) R*_{f}*0.52; FAB-MS *m*/*z* 430 ((M+H)⁺).

### SPECIFIC COMPOUND PREPARATIONS

Descriptions of the detailed preparative steps used to prepare the specific compounds listed in Tables 1-4 are provided below. Many of the compounds listed in the Tables can be synthesized following a variety of methods. The specific examples below are therefore provided by way of illustration only and should not be construed to limit the scope of the invention in any way.

Entry 5: *N*-(4-*tert*-Butylpyridyl)-*N'*-(4-(4-pyridinylmethyl)phenyl urea was prepared according to Method B2a.

Entry 6: 4-*tert*-Butyl-2-aminopyridine was reacted with 4-phenoxyaniline according to Method B2c to afford the urea.

Entry 7: 4-*tert*-Butyl-2-aminopyridine was reacted with 4-(4-methylphenoxy)aniline according to Method B2c to afford the urea.

Entry 8: *N*-(4-*tert*-Butylpyridyl)-*N'*-(4-(4-chlorophenoxy)phenyl) urea was prepared according to Method B2c.

Entry 10: 4-(4-Aminophenoxy)pyridine was prepared starting from 4-hydroxypyridine and 1-bromo-3-nitrobenzene according to Method A3F. 4-*tert*-Butyl-2-aminopyridine was reacted with 4-(4-aminophenoxy)pyridine according to Method B2a to afford the urea.

Entry 11: 4-(4-Pyridylthio)aniline was prepared starting from 4-aminothiophenol and 4-chloropyridine hydrochloride according to Method A4a. 4-*tert*-Butyl-2-arninopyridine was reacted with 4-(4-pyridylthio)aniline according to Method B2c to afford the urea.

Entry 12: 4-(4-Pyridylthio)aniline was prepared starting from 4-aminothiophenol and 4-chloropyridine hydrochloride according to Method A4a. 4-*tert*-Butyl-2-aminopyridine was reacted with 3-(4-pyridylthio)aniline according to Method B2c to afford the urea.

Entry 20: 4-(4-Aminophenoxy)pyridine was prepared starting from 4-hydroxypyridine and 1-bromo-3-nitrobenzene according to Method A3f. 3-Aminoisoquinoline was reacted with 4-(4-aminophenoxy)pyridine according to Method B2a to afford the urea.

Entry 22: *N,N'*-(Bis(3-(2-methoxyquinolinyl)) urea) was prepared according to Method B2b.

Entry 23: 3-Amino-2-methoxyquinoline and 4-(4-pyridylmethyl)aniline were reacted according to Method B3 to afford the urea.

Entry 24: 3-Amino-2-methoxyquinoline was reacted with 4-(4-pyridylcarbonyl)aniline according to Method B4 to afford the urea.

Entry 25: 4-(4-Pyridyloxy)aniline was prepared starting from 4-hydroxypyridine and 1-fluoro-4-nitrobenzene according to Method A3d. 3-Amino-2-methoxyquinoline was reacted with 4-(4-pyridyloxy)aniline according to Method B2c to afford the urea.

Entry 26: 3-Amino-2-methoxyquinoline was reacted with 4-((4-methoxyphenyl)methylamino)aniline according to Method B4 to afford the urea.

Entry 27: 3-(4-Pyridylthio)aniline was prepared according to Method A4a. 3-Amino-2-methoxyquinoline and 3-(4-pyridylmethyl)aniline were reacted according to Method B3 to afford the urea.

Entry 28: 4-(4-Pyridyloxy)aniline was prepared starting from 4-hydroxypyridine and 1-fluoro-4-nitrobenzene according to Method A3d. 1-(4-Methylpiperazinyl)-3-aminoisoquinoline was reacted with 4-(4-aminophenoxy)pyridine according to Method B2a to afford the urea.

The following compounds have been synthesized according to the General Methods listed above:

**Table 1. 4-tert-Butyl-2-pyridyl Ureas**

| | | | | | | |
|---|---|---|---|---|---|---|
| 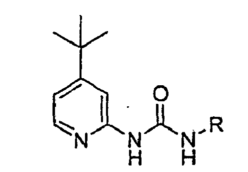 | | | | | | |

| Entry | R | mp (°C) | HPLC (min.) | TLC R*_{f}* | TLC Solvent System | Mass Spec. [Source] |
|---|---|---|---|---|---|---|
| 5 | 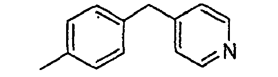 | | | 0.47 | 100% EtOAc | 361 (M+H)+ (FAB) |
| 6 | 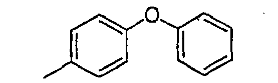 | 179 - 180 | | 0.58 | 5% MeOH / 95% CH2Cl2 | 362 (M+H)+ (FAB) |
| 7 | 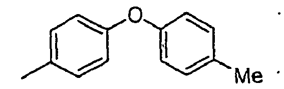 | 190 - 191 | | 0.46 | 5% MeOH / 95% CH2Cl2 | 376 (M+H)+ (FAB) |
| 8 | 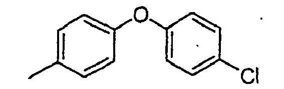 | 198 - 199 | | 0.76 | 5% MeOH / 95% CH2Cl2 | 396 (M+H)+ (FAB) |
| 10 | 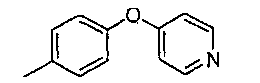 | | | 0.40 | 100% EtOAc | 363 (M+H)+ (FAB) |
| 11 | 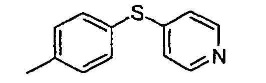 | 208 - 212 | | 0.39 | 5% MeOH / 95% CH2Cl2 | 379 (M+H)+ (HPLC ES-MS) |
| 12 | 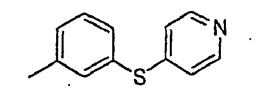 | 196 - 197 | | 0.37 | 5% MeOH / 95% CH2Cl2 | 379 (M+H)+ (FAB) |

**Table 2. 3-Isoquinolyl Ureas**

| | | | | | | |
|---|---|---|---|---|---|---|
| 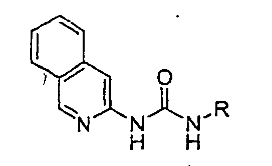 | | | | | | |

| Entry | R | mp (°C) | HPLC (min.) | TLC R*_{f}* | TLC Solvent System | Mass Spec. [Source] |
|---|---|---|---|---|---|---|
| 20 | 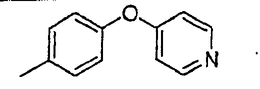 | | | 0.27 | 100% EtOAc | 357 (M+H)+ (FAB) |

**Table 3. 2-Methoxy-3-quinolyl Ureas**

| | | | | | | |
|---|---|---|---|---|---|---|
| 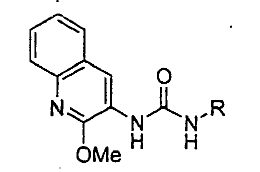 | | | | | | |

| Entry | R | mp (°C) | HPLC (min.) | TLC R*_{f}* | TLC Solvent System | Mass Spec. [Source] |
|---|---|---|---|---|---|---|
| 22 | 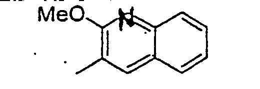 | - | | 0.45 | 50% EtOAc / 50% hexane | 375 (N+H)+ (HPLC ES-MS) |
| 23 | 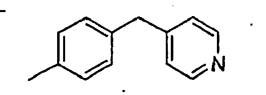 | | | 0.56 | 50% EtOAc / 50% hexane | 385 (M+H)+ (FAB) |
| 24 | 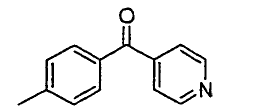 | | | 0.45 | 100% EtOAc | 399 (M+H)+ (FAB) |
| 25 | 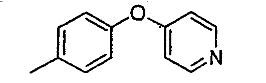 | 207 - 208 | | 0.24 | 5% MeOH / 95% CH2Cl2 | 387 (M+H)+ (FAB) |
| 26 | 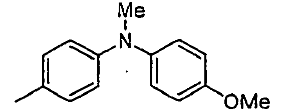 | 126 - 130 | | | | |
| 27 | 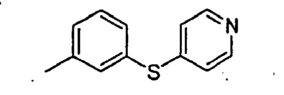 | | | 0.39 | 50% acetone / 50% CH2Cl2 | 403 (M+H)+ (FAB) |

**Table 4. 3-Quinolyl Ureas**

| | | | | | | |
|---|---|---|---|---|---|---|
| 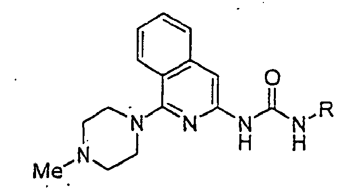 | | | | | | |

| Entry | R | mp (°C) | HPLC (min.) | TLC R*_{f}* | TLC Solvent System | Mass Spec. [Source] |
|---|---|---|---|---|---|---|
| 28 | 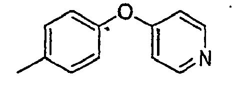 | | | 0.20 | 30% MeOH / 70% EtOAc | 455 (M+H)+ (HPLC ES-MS) |

### BIOLOGICAL EXAMPLES

### In Vitro raf Kinase Assay:

In an in vitro kinase assay, raf was incubated with MEK in 20 mM Tris-HCl, pH 8.2 containing 2 mM 2-mercaptoethanol and 100 mM NaCl. This protein solution (20 µL) was mixed with water (5 µL) or with compounds diluted with distilled water from 10 mM stock solutions of compounds dissolved in DMSO. The kinase reaction was initiated by adding 25 µL [λ-³³P]ATP (1000-3000 dpm/pmol) in 80 mM Tris-HCl, pH 7.5, 120 mM NaCl, 1.6 mM DTT, 16 mM MgCl₂. The reaction mixtures were incubated at 32 °C, usually for 22 min. Incorporation of ³³P into protein was assayed by harvesting the reaction onto phosphocellulose mats, washing away free counts with a 1% phosphoric acid solution and quantitating phosphorylation by liquid scintillation counting. For high throughput screening, 10 µM ATP and 0.4 µM MEK was used. In some experiments, the kinase reaction was stopped by adding an equal amount of Laemmli sample buffer. Samples were boiled 3 min and the proteins resolved by electrophoresis on 7.5% Laemmli gels. Gels were fixed, dried and exposed to an imaging plate (Fuji). Phosphorylation was. analyzed using a Fujix Bio-Imaging Analyzer System.

All compounds exemplified displayed IC₅₀s of between 10 nM and 10 µM.

### Cellular Assay:

For in vitro growth assay, human tumor cell lines, including but not limited to HCT116 and DLD-1, containing mutated K-ras genes were used in standard proliferation assays for anchorage dependent growth on plastic or anchorage independent growth in soft - agar. Human tumor cell lines were obtained from ATCC (Rockville MD) and maintained in RPMI with 10% heat inactivated fetal bovine serum and 200 mM glutamine. Cell culture media and additives were obtained from Gibco/BRL (Gaithersburg, MD) except for fetal bovine serum (JRH Biosciences, Lenexa, KS). In a standard proliferation assay for anchorage dependent growth, 3 X 10³ cells were seeded into 96-well tissue culture plates and allowed to attach overnight at 37 °C in a 5% CO₂ incubator. Compounds were titrated in media in dilution series and added to 96-well cell cultures. Cells were allowed to grow 5 days typically with a feeding of fresh compound containing media on day three. Proliferation was monitored by measuring metabolic activity with standard XTT colorimetric assay (Boehringer Mannheim) measured by standard ELISA plate reader at OD 490/560, or by measuring ³H-thymidine incorporation into DNA following an 8 h culture with 1 µCu ³H-thymidine, harvesting the cells onto glass fiber mats using a cell harvester and measuring ³H-thymidine incorporation by liquid scintillant counting.

For anchorage independent cell growth, cells were plated at 1 x 10³ to 3 x 10³ in 0.4% Seaplaque agarose in RPMI complete media, overlaying a bottom layer containing only 0.64% agar in RPMI complete media in 24-well tissue culture plates. Complete media plus dilution series of compounds were added to wells and incubated at 37 °C in a 5% CO₂ incubator for 10-14 days with repeated feedings of fresh media containing compound at 3-4 day intervals. Colony formation was monitored and total cell mass, average colony size and number of colonies were quantitated using image capture technology and image analysis software (Image Pro Plus, media Cybernetics).

These assays established that the compounds of formula I are active to inhibit raf kinase activity and to inhibit oncogenic cell growth.

### In Vivo Assay:

An in vivo assay of the inhibitory effect of the compounds on tumors (e.g., solid cancers) mediated by raf kinase can be performed as follows:

CDI nu/nu mice (6-8 weeks old) are injected subcutaneously into the flank at 1 x 10⁶ cells with human colon adenocarcinoma cell line. The mice are dosed i.p., i.v. or p.o. at 10, 30, 100, or 300 mg/Kg beginning on approximately day 10, when tumor size is between 50-100 mg. Animals are dosed for 14 consecutive days once a day; tumor size was monitored with calipers twice a week.

The inhibitory effect of the compounds on raf kinase and therefore on tumors (e.g., solid cancers) mediated by raf kinase can further be demonstrated in vivo according to the technique of Monia et al. (*Nat. Med.* **1996**, *2,* 668-75).

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to.various usages and conditions.

## Claims

1. A compound of the following formula :
A'-D-B' (II)
or a pharmaceutically acceptable salt thereof, wherein
D is -NH-C(O)-NH-,
A' is a substituted isoquinolinyl group or unsubstituted isoquinolinyl group or an unsubstituted quinolinyl group,
B' is a substituted or unsubstituted bridged cyclic structure of up to 30 carbon atoms of the formula -L-(ML¹)_{q} wherein L comprises a cyclic moiety having at least 5 members and is bound directly to D, L' comprises a cyclic moiety having at least 5 members, M is selected from the group consisting of -O-, -S-, -N(R⁷)-, -(CH₂)ₘ-, -C(O)-, -CH(OH)-, -(CH₂)ₘO-, -(CH₂)ₘS-, -(CH₂)ₘ N(R⁷)-, -O(CH₂)ₘ- ; -CHX^{a}-, -CX^{a}₂-, -S-(CH₂)ₘ- and -N(R⁷)(CH₂)ₘ-, where m=1-3, X^{a} is halogen, and R⁷ is as defined below, q is an integer of from 1-3, and each cyclic structure of L and L¹ contains 0-4 members of the group consisting of nitrogen, oxygen and sulfur, subject to the proviso that B' is not 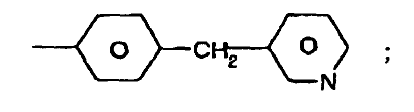 or 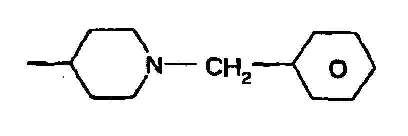
wherein the substituents for the substituted isoquinolinyl groups of A' are selected from the group consisting of halogen, up to per-halo, and Wn, where n is 0-3 and each W is independently selected from the group consisting of C₁₋₁₀ alkyl, C₁₋₁₀ alkoxy, at least a five membered C₃₋₁₀ cycloalkyl having 0-3 heteroatoms, C₂₋₁₀ alkenyl, C₁₋₁₀ alkenoyl, substituted C₁₋₁₀ alkyl, substituted C₁₋₁₀ alkoxy, at least a five-membered substituted C₃₋₁₀ cycloalkyl having 0-3 heteroatoms selected from N, S and O; -CN, up to per halo substituted C₆-C₁₄ aryl, up to per halo substituted C₇-C₂₄ alkaryl, up to per halo substituted C₇-C₂₄ aralkyl, up to per halo substituted C₃-C₁₂ heteroaryl having at least 5 members and 1-3 heteratoms selected from O, N and 5, up to per halo substituted C₄-C₂₄ alkheteroaryl having at least 5 members and 1-3 heteroatoms selected from O, N and S, C₆-C₁₄ aryl, C₁-C₂₄ alkaryl, C₁-C₂₄ aralkyl, C₃-C₁₂ heteroaryl having at least 5 cyclic members and 1-3 heteroatoms selected from O, N and S, C₄-C₂₄ alkheteroaryl having at least 5 cyclic members and 1-3 heteroatoms selected from O, N and S; -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstltuted C₂₋₁₀ alkenyl and up to per halosubstituted C₁₋₁₀ alkenoyl;
where B' is substituted, the substituents are selected from the group consisting of halogen, up to per-halo, and In, where n is 0-3 and each J is independently selected from the group consisting of -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O) R^{7'}, with each R⁷ and R^{7'} independently as defined above for W, C₁₋₁₀ alkyl, C₁₋₁₀ alkoxy, at least a five-membered C₃-C₁₀ cycloalkyl having 0-3 heteroatoms, C₂-C₁₀ alkenyl, C₁-C₁₀ alkenoyl, C₆₋₁₂ aryl, at least a five-membered C₃₋₁₂ hetaryl having 1-3 heteroatoms selected from N, S and O, C₇₋₂₄ aralkyl, C₇₋₂₄ alkaryl, substituted C₁₋₁₀ alkyl, substituted C₁₋₁₀ alkoxy, at least a five-membered substituted C₃₋₁₀ cydoalkyl having 0-3 heteroatoms selected from N, S and O, substituted C₆ - C₁₄ aryl, at least a J-membered substituted C₃₋₁₂ hetaryl having 1-3 heteroatoms selected from N, S and O, substituted C₇₋₂₄ alkaryl and substituted C₇-C₂₄ aralkyl,
subject to the proviso that where B' is -L(ML¹)_{q}, L¹ is not substituted by the substituents -C(O)R^{a}, -C(NR^{a})R^{b}, -C(O)NR^{a}R^{b} and -SO₂R^{a} wherein each R^{a} and R^{b} are independently hydrogen or a carbon based moiety of up to 24 carbon atoms, optionally containing heteroatoms selected from N, S and O,
where J is a substituted group, it is substituted by halogen, up to per halo, or by one or more substituents independently selected from the group consisting of -CN, -CO₂R⁷, -OR⁷, -SR⁷, -NR⁷R^{7'}, -NO₂, -NR⁷C(O)R^{7'} and -NR⁷C(O)OR^{7'} ; with each R⁷ and R^{7'} independently as defined above for W.

2. A compound as in dalm 1, wherein :
R^{a} and R^{b} are, independently, C₁₋₁₀ alkyl, C₁₋₁₀ alkoxy, C₃₋₁₀ cycloalkyl having 0-3 heteroatoms, C₂₋₁₀ alkenyl, C₁₋₁₀ alkenoyl, C₆₋₁₂ aryl, C₃₋₁₂ hetaryl having 1-3 heteroatoms selected from N, S and O, C₇₋₂₄ aralkyl, C₇₋₂₄ alkaryl, substituted C₁₋₁₀ alkyl, substituted C₁₋₁₀ alkoxy, substituted C₃₋₁₀ cycloalkyl having 0-3 heteroatoms selected from N, S and 0, substituted C₆ -C₁₄ aryl, substituted C₃₋₁₂ hetaryl having 1-3 heteroatoms selected from N, S and O, substituted C₇₋₂₄ alkaryl or substituted C₇-C₂₄ aralkyl, where R^{a} is a substituted group, it is substituted by halogen up to per halo.

3. A compound of daim 1 or 2, wherein L in the formula -L-(ML¹)_{q} for B' is a substituted 6 member cyclic aryl moiety, a substituted 5-6 member cyclic hetaryl moiety, an unsubstituted 6 member cyclic aryl moiety, or an unsubstituted 5-6 member cyclic hetaryl moiety, and L¹ in the formula -L-(ML¹)_{q} of B' is a substituted aryl moiety having at least six cyclic members, an unsubstituted aryl moiety having at least six cyclic members, a substituted hetaryl moiety having at least 5 cyclic members or an unsubstituted hetaryl moiety having at least 5 cydic members, said hetaryl moieties having 1 to 4 members selected from the group of hetero atoms consisting of nitrogen, oxygen and sulfur with the balance of the hetaryl moiety being carbon.

4. A compound of any one of claims 1 to 3, wherein L and L' are each Independently selected from the group consisting of thiophene, phenyl, substituted phenyl, pyridinyl, substituted pyridinyl, pyrimidinyl, substituted pyrimidinyl, napthyl, substituted napthyl, quinolinyl, substituted quinolinyl, isoquinodiyl and substituted isoquinodiyl.

5. A compound of any one of claims 1 to 4, wherein B' is a substituted group, it is substituted by -CN, halogen, C₁₋₁₀ alkyl, C₁₋₁₀ alkoxy, -OH, up to per halo substituted C₁₋₁₀ alkyl, up to per halo substituted C₁₋₁₀ alkoxy, -O(R⁷), -SR⁷, -NR⁷R^{7'} or -NO₂, wherein each R⁷ and R^{7'} are independendy selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁-₁₀ alkoxy, up to per halosubstituted C₂₋₁₀ alkenyl and up to per halosubstituted C₁₋₁₀ alkenoyl,

6. A compound of any one of claims 1 to 5, wherein M in the formula -L-(ML¹) for B' is -O-, -CH₂-, -S-, -NH-, -C(O)-, -O-CH₂ or -CH₂-O-.

7. A compound of the formula A'-D-B', or a pharmaceutically acceptable salt thereof, wherein D is -NH-C(O)-NH-
A' is a substituted isoquinolinyl group or unsubstituted isoquinolinyl group or an unsubstituted quinolinyl group,
B' is of the formula -L-(ML¹)_{q}, wherein L is phenyl or substituted phenyl and L¹ is phenyl, substituted phenyl, pyridinyl or substituted pyridinyl, q is an integer of from 1-2 and M is selected from the group consisting of -O-, -S-, -N(R⁷)-, -(CH₂)ₘ-, -C(O)-, -CH(OH)-, -(CH₂)ₘO-, -(CH₂)ₘS-, -(CH₂)ₘN(R⁷)-, -O(CH₂)ₘ- ; -CHX^{a}-, -CX^{a}₂-, -S-(CH₂)ₘ- and -N(R⁷)(CH₂)ₘ-, where m=1-3, X^{a} is halogen, and R⁷ is as defined below, subject to the proviso that B' is not 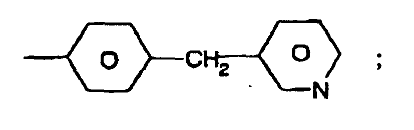
wherein the substituents for the substituted isoquinolinyl groups of A' are selected from the group consisting of halogen, up to per-halo, and wn,
where n is 0-3 and each W is independently selected from the group consisting of C₁₋₁₀ alkyl, C₁₋₁₀ alkoxy, at least a five membered C₃₋₁₀ cycloalkyl having 0-3 heteroatoms, C₂₋₁₀ alkenyl, C₁₋₁₀ alkenoyl, substituted C₁₋₁₀ alkyl, substituted C₁₋₁₀ alkoxy, at least a five membered substituted C₃₋₁₀ cycloalkyl having 0-3 heteroatoms selected from N, S and O; -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halusubstituted C₁₋₁₀ alkoxy, up to per halosubstituted C₂₋₁₀ alkenyl and up to per halosubstituted C₁₋₁₀ alkenoyl;
wherein B' is substituted, the substituents are selected from the group consisting of halogen, up to per-halo, and Jn, where n is 0-3 and each J is independently selected from the group consisting of -CN, -NO₂, -OR⁷, -SR⁷, -NR⁷R^{7'}, -NR⁷C(O)OR^{7'}, -NR⁷C(O)R^{7'}, with each R⁷ and R^{7'} independently as defined above for W, C₁₋₁₀ alkyl, C₁₋₁₀ alkoxy, at least a five membered C₃₋₁₀ cycloalkyl having 0-3 heteroatoms, C₂₋₁₀ alkenyl, C₁₋₁₀ alkenoyl, C₆₋₁₂ aryl, at least a five membered C₃₋₁₂ hetaryl having 1-3 heteroatoms selected from N, S and O, C₇₋₂₄ aralkyl, C₇₋₂₄ alkaryl, substituted C₁₋₁₀ alkyl, substituted C₁₋₁₀ alkoxy, at least a five membered substituted C₃₋₁₀ cycloalkyl having 0-3 heteroatoms selected from N, S and O, substituted C₆ -C₁₄ aryl, at least a five membered substituted C₃₋₁₂ hetaryl having 1-3 heteroatoms selected from N, S and O, substituted C₇₋₂₄ alkaryl and substituted C₇-C₂₄ aralkyl,
subject to the proviso that L¹ is not substituted by the substituents -C(O)R^{a}, -C(NR^{a})R^{b}, -C(O)NR^{a}R^{b} and -SO₂R^{a} wherein R^{a} and R^{b} are each independently, hydrogen or a carbon based moiety of up to 24 carbon atoms, optionally containing heteroatoms selected from N, 5 and O.

8. A compound of claim 7, wherein the substituents for B' are selected from the group consisting of -CN, halogen, C₁₋₁₀ alkyl, C₁₋₁₀ alkoxy, -OH, up to per halo substituted C₁₋₁₀ alkyl, and up to per halo substituted C₁₋₁₀ alkoxy.

9. A compound of any one of claims 1 to 8, wherein the substituted isoquinolinyls of A' have 1-3 substituents selected from the group consisting of C₁₋₁₀ alkyl, up to per halo substituted C₁₋₁₀ alkyl, -CN, -OH, halogen, C₁₋₁₀ alkoxy, up to per halo substituted C₁₋₁₀ alkoxy and at least five-membered C₃-C₁₀ heterocyclic moieties comprising 1 to 2 heteroatoms selected from the group of consisting of nitrogen, oxygen and sulfur.

10. A compound of claim 7 or 8, wherein the substituted Isoquinolinyls of A' have 1-3 substituents selected from the group consisting of C₁₋₁₀ alkyl, up to per halo substituted C₁₋₁₀ alkyl, -CN, -OH, halogen, C₁₋₁₀ alkoxy, up to per halo substituted C₁₋₁₀ alkoxy and at least a five membered C₃₋₁₀ neterocyclic moieties comprising 1 to 2 heteroatoms selected from the group of consisting of nitrogen, oxygen and sulfur.

11. A compound of any one of claims 1 to 10, wherein L and L¹ are independently phenyl, substituted phenyl, pyridinyl, substituted pyridinyl, pyrimidinyl or substituted pyrimidinyl.

12. A compound of any one of claims 1 to 11, wherein L¹ is substituted 1 to 3 times by one or more substituents selected from the group consisting of C₁-C₁₀ alkyl, up to per halo substituted C₁-C₁₀ alkyl, -CN, -OH, halogen, C₁-C₁₀ alkoxy and up to per halo substituted C₁-C₁₀ alkoxy.

13. A compound of any one of claims 7, 8 and 10, wherein L¹ is substituted 1 to 3 times by one or more substituents selected from the group consisting of C₁-C₁₀ alkyl, up to per halo substituted C₁-C₁₀ alkyl, -CN, -OH, halogen, C₁-C₁₀ alkoxy and up to per halo substituted C₁-C₁₀ alkoxy.

14. A compound of claim 1 wherein each substituent J is independently selected from the group consisting of C₁-C₁₀ alkyl, up to per halo substituted C₁-C₁₀ alkyl, -CN, -OH, halogen, C₁-C₁₀ alkoxy , up to per halo substituted C₁-C₁₀ alkoxy, -CN, -CO₂ R⁷, -C(O)N R⁷ R^{7'}, -C(O)- R⁷, -NO₂, -O R⁷, -S R⁷, -N R⁷ R^{7'}, -N R⁷C(O)O R^{7'} and -N R⁷C(O) R^{7'}, where R⁷ and R^{7'} are each, independently, as defined for W in claim 1,
M is -0-, -S-, -N(R⁷)-, -(CH₂)ₘ-, -C(O)-, -CH(OH)-, -(CH₂)ₘO- and -O(CH₂)ₘ-, where m= 1-3; and
L¹ is phenyl, pyridinyl, pyrimidinyl, substituted phenyl, substituted pyridinyl and substituted pyrimidinyl, with substituents selected from the group consisting of -CN; -OH; halogen up to per-halo; C₁-C₁₀ alkoxy and halosubstituted C₁-C₁₀ alkoxy, up to per-halo.

15. A compound of any one of daims 1 to 14, which is a pharmaceutically acceptable salt of a compound of formula II selected from the group consisting of
a) basic salts of organic acids and inorganic acids selected from the group consisting of hydrochloric add, hydrobromic acid, sulfuric acid, phosphoric acid, methanesulfonic acid, trifluorosulfonic acid, benzenesulfonic add, p-toluene sulfonic acid (tosylate salt), 1-napthalene sulfonic acid, 2-napthalene sulfonic acid, acetic acid, sulfonic acid, 2-napthalene sulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid; and
b) acid salts of organic and inorganic bases containing cations selected from the group consisting of alkaline cations, alkaline earth cations, the ammonium cation, aliphatic substituted ammonium cations and aromatic substituted ammonium cations.

16. A compound of claim 7 which is a pharmaceutically acceptable salt of a compound of formula II selected from the group consisting of
a) basic salts of organic acids and inorganic acids selected from the group consisting of hydrochloric acid, hydrobromic acid, sulphuric acid, phosphoric acid, methanesulphonic acid, trifluorosulphonic acid, benzenesulfonic acid, p-toluene sulfonic acid (tosylate salt), 1-napthalene sulfonic acid, 2-napthalene sulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid; and
b) acid salts of organic and inorganic bases containing cations selected from the group consisting of alkaline cations, alkaline earth cations, the ammonium cation, aliphatic substituted ammonium cations and aromatic substituted ammonium cations.

17. A pharmaceutical composition comprising a compound of claim 1 and a physiologically acceptable carrier.

18. A pharmaceutical composition comprising a compound of claim 7 and a physiologically acceptable carrier.

19. A compound selected from the group consisting of :
N-(3-isoquinolinyl)-N'-(4-(4-pyridinyloxy)phenyl) urea;
N-(1-(4-methylpiperazinyl)-3-isoquinolinyl)-N' (4-(4-pyridinyloxy)phenyl) urea;
or a pharmaceutically acceptable salt thereof.

20. A pharmaceutical composition comprising a compound of claim 19 and a physiologically acceptable carrier.

21. A compound of claim 1 of one of the following formulae 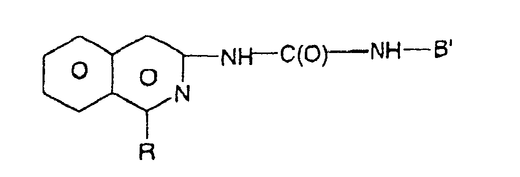 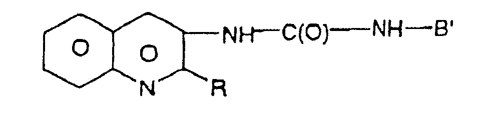 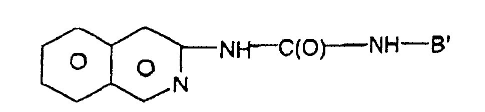 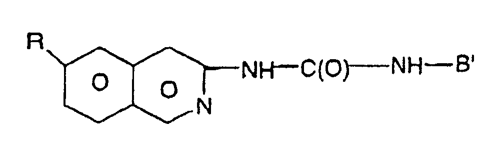 wherein B' is as defined in claim 1 and R is selected from the group consisting of halogen, C₁₋₁₀ alkyl, C₁₋₁₀ alkoxy, C₂₋₁₀ alkenyl, C₁₋₁₀ alkenoyl, -CN, -CO₂R⁷, -C(O)NR⁷R^{7'}, -C(O)-R⁷, -NO₂, -OR⁷, -SR⁷, -NR⁷R^{7'}, -NR⁷((O)OR^{7'}, -NR⁷C(O)R^{7'}, with each R⁷ and R^{7'} are independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl.

22. A compound of claim 1, wherein L is :
(i) phenyl, optionally substituted with 1-3 substituents independently selected from the group consisting of R⁷, OR⁷, NR⁷R^{7'}, C(O)R⁷, C(O)OR⁷, C(O)NR⁷R^{7'}, NR⁷C(O)R^{7'}, NR⁷C(O)OR^{7'}, halogen, cyano and nitro; or
(ii) pyridyl, optionally substituted with 1-3 substituents Independently selected from the group consisting of R⁷, OR⁷, NR⁷R^{7'}, C(O)R⁷, C(O)OR⁷, C(O)NR⁷R^{7'}, NR⁷C(O)R^{7'}, NR⁷C(O)OR^{7'}, halogen, cyano, and nitro; or
(ii) pyrimidinyl, optionally substituted with 1-3 substituents independently selected from the group consisting of R⁷, OR⁷, NR⁷R^{7'}, C(O)R⁷, C(O)OR⁷, C(O)NR⁷R^{7'}, NR⁷C(O)R^{7'}, NR⁷C(O)OR^{7'}, halogen, cyano and nitro,

23. A compound of claim 1, wherein L¹ is phenyl, pyridinyl or pyrimidinyl.

24. A compound of claim 1
where L is
(I) phenyl, optionally substituted with 1-3 substituents independently selected from the group consisting of C₁-C₅ linear or branched alkyl, C₁-C₅ linear or branched haloalkyl, C₁-C₃ alkoxy, hydroxy, amino, C₁-C₃ alkylamino, C₁-C₆ dialkylamino, halogen, cyano, and nitro; or
(II) pyridyl, optionally substituted with 1-3 substituents Independently selected from the group consisting of C₁-C₅ linear or branched alkyl, C₁-C₅ linear or branched haloalkyl, C₁-C₃ alkoxy, hydroxy, amino, C₁-C₃ alkylamino, C₁-C₆ dialkylamino, halogen, cyano, and nitro; and
L¹ comprises a cyclic moiety selected from the group consisting of:
(ii) naphthyl, optionally substituted with 1-3 substituents independently selected from the group consisting of R⁷, OR⁷, NR⁷R^{7'}, NR⁷C(O)R^{7'}, NR⁷C(O)OR^{7'}, halogen, cyano and nitro;
(iii) pyridyl, optionally substituted with 1-3 substituents independently selected from the group consisting of R⁷, OR⁷, NR⁷R^{7'}, NR⁷C(O)R^{7'}, NR⁷C(O)OR^{7'}, halogen, cyano and nitro;
(iv) pyrimidinyl, optionally substituted with 1-3 substituents independently selected from the group consisting of R⁷, OR⁷, NR⁷R^{7'}, NR⁷C(O)R^{7'}, NR⁷C(O)OR^{7'}, halogen, cyano and nitro;
(v) quinolinyl optionally substituted with 1-3 substituents independently selected from the group consisting of R⁷, OR⁷, NR⁷R^{7'}, NR⁷C(O)R^{7'}, NR⁷C(O)OR^{7'}, halogen, cyano and nitro;
(vi) isoquinolinyl optionally substituted with 1-3 substituents independently selected from the group consisting of R⁷, OR⁷, NR⁷R^{7'}, NR⁷C(O)R^{7'}, NR⁷C(O)OR^{7'}, halogen, cyano and nitro; and each R⁷ and R^{7'} is independently
(a) hydrogen,
(b) C₁-C₆ linear, branched, or cyclic alkyl, optionally substituted with 1-3 substituents independently selected from the group consisting of C₁-C₅ linear or branched alkyl, up to perhalo substituted C₁-C₅ linear or branched alkyl, C₁-C₃ alkoxy and hydroxy;
(c) C₁-C₆ alkoxy, optionally substituted with 1-3 substituents independently selected from the group consisting of C₁-C₅ linear or branched alkyl, up to perhalo substituted C₁-C₅ linear or branched alkyl, C₁-C₃ alkoxy, hydroxy and halogen;
(d) phenyl, optionally substituted with 1-3 substituents independently selected from the group consisting of C₁-C₅ linear or branched alkyl, up to perhalo substituted C₁-C₅ linear or branched alkyl, C₁-C₃ alkoxy, hydroxy and halogen, '
(e) 5-6 membered monocydic heteroaryl having 1-4 heteroatoms selected from the group consisting of 0, N and S or 8-10 membered bicyclic heteroaryl having 1-6 heteroatoms selected from the group consisting of 0, N and S, optionally substituted with 1-3 substituents independently selected from the group consisting of C₁-C₅ linear or branched alkyl, up to perhalo substituted C₁-C₅ linear or branched alkyl, C₁-C₃ alkoxy, hydroxy and halogen,
(f) C₁-C₃ alkyl-phenyl, optionally substituted with 1-3 substituents independently selected from the group consisting of C₁-C₅ linear or branched alkyl, up to perhalo substituted C₁-C₅ linear or branched alkyl, C₁-C₃ alkoxy, hydroxy and halogen; and
(g) up to per-halo substituted C₁-C₅ linear, branched or cyclic alkyl, and where not per-halo substituted, optionally substituted with 1-3 substituents independently selected from the group consisting of C₁-C₅ linear or branched alkyl, up to perhalo substituted C₁-C₅ linear or branched alkyl, C₁-C₃ alkoxy and hydroxy.

25. a compound as in claim 1 wherein A', L and L¹ follow one of the following of combinations:
A'= isoquinolinyl, L=phenyl and L¹ is phenyl, pyridinyl, quinolinyl or isoquinolinyl,
A'= quinolinyl, L=phenyl and L¹ is phenyl, pyridinyl, quinolinyl or isoquinolinyl,
A'= quinolinyl, L=pyridinyll and L¹ is phenyl, pyridinyl, quinolinyl or isoquinolinyl,
A'= substituted quinolinyl, L=phenyl and L¹= is phenyl, pyridinyl, quinolinyl or isoquinolinyl, or
A'= substituted quinolinyl, L=pyridinyl and L¹= is phenyl, pyridinyl, quinolinyl or isoquinolinyl.

26. A compound of claim 1 wherein L¹ is pyridyl.

27. Use of a compound according to any one of claims 1 to 26 for the preparation of a medicament for the treatment of a condition where inhibition of the raf kinase pathway is needed.

28. Use of a compound according to any one of claims 1 to 26, for the preparation of a medicament for the treatment of tumors and/or cancerous cell growth mediated by raf kinase.

29. Use according ti claim 27 or 28, wherein said condition is a murine cancer, solid cancers, such as, for example, carcinomas, for example of the lungs, pancreas, thyroid, bladder or colon, myeloid disorders, for example myeloid leukaemia, or adenomas, for example villous colon adanoma.

## Patentansprüche

1. Verbindung aus der nachfolgenden Formel:
A'-D-B' (II)
oder ein pharmazeutisch unbedenkliches Salz davon, wobei
D -NH-C(O)-NH-
ist,
A' eine substituierte Isoquinolinyl-Gruppe oder eine nichtsubstituierte Isoquionolinyl-Gruppe oder eine nichtsubtituierte Quinolinyl-Gruppe ist,
B' eine substituierte oder nichtsubstituierte zyklische Struktur aus bis zu 30 Kohlenstoffatomen der Formel -L-(ML¹)_{q} umfasst, wobei L ein zyklischer Rest mit mindestens 5 Gliedern und direkt an D gebunden ist, L' einen zyklischen Rest mit mindestens 5 Gliedern umfasst, M ausgewählt ist aus der Gruppe bestehend aus -O-, -S-, -N(R⁷)-, -(CH₂)ₘ-, -C(O)-, -CH(OH)-, -(CH₂)ₘO-, -(CH₂)ₘS-, -(CH₂)ₘ, N(R⁷)-, -O(CH₂)ₘ-; -CHX^{a}-, -CX^{a}₂-, -S-(CH₂)ₘ-, und -N(R⁷)(CH₂)ₘ-, wobei m=1-3, X^{a} ein Halogen und R⁷ wie nachstehend definiert ist, q eine ganze Zahl von 1-3 ist und jede zyklische Struktur von L ist und L¹ 0-4 Glieder der Gruppe bestehend aus Stickstoff, Sauerstoff und Schwefel ist, vorausgesetzt, dass B' nicht, 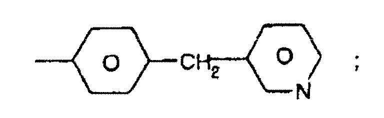 oder 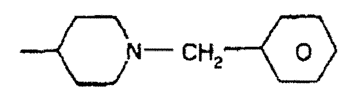
ist, wobei die Substituenten der substituierten Isoquinolinyl-Gruppen von A' ausgewählt sind aus der Gruppe bestehend aus Halogen, bis zu per-Halo und Wn, wobei n 0-3 ist und jedes W unabhängig ausgewählt ist aus der Gruppe bestehend aus C₁₋₁₀ Alkyl, C₁₋₁₀ Alkoxy, wenigstens einem fünfgliedrigen C₃₋₁₀ Cycloalkyl mit 0-3 Heteroatomen, C₂₋₁₀ Alkenyl, C₁₋₁₀ Alkenoyl, substituiertem C₁₋₁₀ Alkyl, substituiertem C₁₋₁₀ Alkoxy, wenigstens einem fünfgliedrigen substituierten C₃₋₁₀ Cycloalkyl mit 0-3 Heteroatomen, ausgewählt aus N, S und O; bis zu per-Halo substituiertem C₆-C₁₄ Aryl, bis zu per-Halo substituiertem C₇-C₂₄ Alkaryl, bis zu per-Halo substituiertem C₇-C₂₄ Aralkyl, bis zu per-Halo substituiertem C₃-C₁₂ Heteroaryl mit wenigstens 5 Gliedern und 1-3 Heteroatomen, ausgewählt aus O, N und S, bis zu per-Halo substituiertem C₄-C₂₄ Alkheteroaryl mit wenigstens 5 Gliedern und 1-3 Heteroatomen, ausgewählt aus O, N und S, C₆-C₁₄ Aryl, C₁-C₂₄ Alkaryl, C₁-C₂₄ Aralkyl, C₃-C₂ Heteroaryl mit wenigstens 5 zyklischen Gliedern und 1-3 Heteroatomen, ausgewählt aus O, N und S, C₄-C₂₄ Alkheteroaryl mit wenigstens 5 zyklischen Gliedern und 1-3 Heteroatomen, ausgewählt aus O, N und S; -CO₂R⁷, -C(O)NR⁷R^{7'}, -C(O)-R⁷, -NO₂, -OR⁷, -SR^{7,} -NR⁷R^{7'}, -NR⁷C(O)OR^{7'}, -NR⁷C(O)R^{7'}, wobei jedes R⁷ und R^{7'} unabhängig ausgewählt ist aus Wasserstoff, C₁₋₁₀ Alkyl, C₁₋₁₀ Alkoxy, C₂₋₁₀ Alkenyl, C₁₋₁₀ Alkenoyl, bis zu per-Halo substituiertem C₁₋₁₀ Alkyl, bis zu per-Halo substituiertem C₁₋₁₀ Alkoxy, bis zu per-Halo substituiertem C₂₋₁₀ Alkenyl und bis zu per-Halo substituiertem C₁₋₁₀ Alkenoyl;
wobei B' substituiert ist, die Substituenten ausgewählt sind aus der Gruppe bestehend aus Halogen, bis zu per-Halo und Jn, wobei n 0-3 ist und jedes J unabhängig ausgewählt ist aus der Gruppe bestehend aus -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O) R^{7'}, wobei jedes R⁷ und R^{7'}, wie oben für W definiert unabhängig ist, C₁₋₁₀ Alkyl, C₁₋₁₀ Alkoxy, wenigstens ein fünfgliedriges C₃-C₁₀ Cycloalkyl mit 0-3 Heteroatomen, C₂-C₁₀ Alkenyl, C₁-C₁₀ Alkenoyl, C₆₋₁₂ Aryl, wenigstens einem fünfgliedrigen C₃₋₁₂ Hetaryl mit 1-3 Heteroatomen, ausgewählt aus N, S und O, C₇₋₂₄ Aralkyl, C₇₋₂₄ Alkaryl, substituiertem C₁₋₁₀ Alkyl, substituiertem C₁₋₁₀ Alkoxy, wenigstens einem fünfgliedrigem substituierten C₃₋₁₀ Cycloalkyl mit 0-3 Heteroatomen, ausgewählt aus N, S und O, substituiertem C₆-C₁₄ Aryl, wenigstens einem J-gliedrigen substituierten C₃₋₁₂ Hetaryl mit 1-3 Heteroatomen, ausgewählt aus N, S und O, substituiertem C₇₋₂₄ Alkaryl und substituiertem C₇-C₂₄ Aralkyl,
vorausgesetzt, dass wenn B' -L(ML¹)_{q} ist, ist L¹ nicht durch die Substituenten -C(O)R^{a}, -C(NR^{a})R^{b}, -C(O)NR^{a}R^{b} und -SO₂R^{a} substituiert, wobei jedes R^{a} und R^{b} unabhängig Wasserstoff oder ein auf Kohlenstoff -basierender Rest von bis zu 24 Kohlenstoffatomen ist, wahlweise enthaltend Heteroatome, ausgewählt aus N, S und O,
wenn J eine substituierte Gruppe ist, dieses durch Halogen substituiert ist, bis zu per-Halo oder durch einen oder mehrere Substituenten, die unabhängig ausgewählt sind aus der Gruppe bestehend aus -CN, -CO₂R⁷, -OR⁷, -NR⁷R^{7'}, -NO₂, -NR⁷C(O)R^{7'} und -NR⁷C(O)OR^{7'}, wobei jedes R⁷ und R^{7'} wie oben für W definiert unabhängig ist.

2. Verbindung nach Anspruch 1, wobei:
R^{a} und R^{b} jeweils unabhängig C₁₋₁₀ Alkyl, C₁₋₁₀ Alkoxy, C₃₋₁₀ Cycloalkyl mit 0-3 Heteroatomen, C₂₋₁₀ Alkenyl, C₁₋₁₀ Alkenoyl, C₆₋₁₂ Aryl, C₃₋₁₂ Hetaryl mit 1-3 Heteroatomen, ausgewählt aus N, S und O, C₇₋₂₄ Aralkyl, C₇₋₂₄ Alkaryl, substituiertes C₁₋₁₀ Alkyl, substituiertes C₁₋₁₀ Alkoxy, substituiertes C₃₋₁₀ Cycloalkyl mit 0-3 Heteroatomen, ausgewählt aus N, S und O, substituiertes C₆-C₁₄ Aryl, substituiertes C₃₋₁₂ Hetaryl mit 1-3 Heteroatomen, ausgewählt aus N, S und O, substituiertes C₇₋₂₄ Alkaryl oder substituiertes C₇-C₂₄ Aralkyl ist, wobei wenn R^{a} eine substituierte Gruppe ist, diese durch Halogen bis zu per-Halo substituiert ist.

3. Verbindung nach Anspruch 1 oder 2, wobei L in der Formel -L-(ML¹)_{q} für B' ein substituierter sechsgliedriger zyklischer Arylrest, ein substituierter fünf- bis sechsgliedriger zyklischer Hetarylrest, ein nichtsubstituierter sechsgliedriger zyklischer Arylrest oder ein nichtsubstituierter fünf- bis sechsgliedriger zyklischer Hetarylrest ist und L¹ in der Formel -L-(ML¹)_{q} von B' ein substituierter Arylrest mit wenigstens sechs zyklischen Gliedern, ein nichtsubstituierter Arylrest mit wenigstens sechs zyklischen Gliedern, ein substituierter Hetarylrest mit wenigstens fünf zyklischen Gliedern oder ein nichtsubstituierter Hetarylrest mit wenigstens fünf zyklischen Gliedern ist, wobei die genannten Hetarylreste 1 bis 4 Glieder aufweisen, die ausgewählt sind aus der Gruppe von Heteroatomen bestehend aus Stickstoff, Sauerstoff und Schwefel, wobei die Differenz des Hetarylrests Kohlenstoff ist.

4. Verbindung nach einem der Ansprüche 1 bis 3, wobei L und L' jeweils unabhängig ausgewählt sind aus der Gruppe bestehend aus Thiophen, Phenyl, substituiertem Phenyl, Pyridinyl, substituiertem Pyridinyl, Pyrimidinyl, substituiertem Pyrimidinyl, Napthyl, substituiertem Napthyl, Quinolinyl, substituiertem Quinolinyl, Isoquinodiyl und substituiertem Isoquinodiyl.

5. Verbindung nach einem der Ansprüche 1 bis 4, wobei B' eine substituierte Gruppe ist, substituiert durch -CN, Halogen, C₁₋₁₀ Alkyl, C₁₋₁₀ Alkoxy, -OH, bis zu per-Halo substituiertem C₁₋₁₀ Alkyl, bis zu per-Halo substituiertem C₁₋₁₀ Alkoxy, -O(R⁷), -SR⁷, -NR⁷R^{7'} oder -NO₂, wobei jedes R⁷ und R^{7'} unabhängig ausgewählt ist aus Wasserstoff, C₁₋₁₀ Alkyl, C₁₋₁₀ Alkoxy, C₂₋₁₀ Alkenyl, C₁₋₁₀ Alkenoyl, bis zu per Halo substituiertem C₁₋₁₀ Alkyl, bis zu per-Halo substituiertem C₁₋₁₀ Alkoxy, bis zu per-Halo substituiertem C₂₋₁₀ Alkenyl und bis zu per-Halo substituiertem C₁₋₁₀ alkenoyl.

6. Verbindung nach einem der Ansprüche 1 bis 5, wobei M in der Formel -1-(ML¹) für B' -O-, -CH₂-, -S-, -NH-, -C(O)-, -O-CH₂- oder -CH₂-O- steht.

7. Verbindung der Formel A'-D-B' oder ein pharmazeutisch unbedenkliches Salz davon, wobei D -NH-C(O)-NH- ist,
A' eine substituierte Isoquinolinyl-Gruppe oder eine nichtsubstituierte Isoquinolinyl-Gruppe oder eine nichtsubstituierte Quinolinyl-Gruppe ist,
B' von der Formel -L-(ML¹)_{q} ist, wobei L Phenyl oder substituiertes Phenyl ist und L¹ Phenyl, substituiertes Phenyl, Pyridinyl oder substituiertes Pyridinyl ist, q eine ganze Zahl von 1-2 ist und M ausgewählt ist aus der Gruppe bestehend aus -O-, -S-, -N(R⁷)-, -(CH₂)ₘ-, -C(O)-, -CH(OH)-, -(CH₂)ₘO-, -(CH)ₘS-, -(CH₂)ₘN(R⁷)-, -O(CH₂)ₘ-; -CHX^{a}-, -CX^{a}₂-, -S-(CH₂)ₘ-, und -N(R⁷)(CH₂)ₘ-, wobei m=1-3, X^{a} ein Halogen und R⁷ wie nachfolgend definiert ist, vorausgesetzt, dass B' nicht 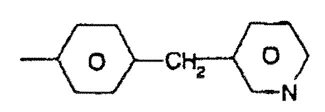 ist;
wobei die Substituenten für die substituierten Isoquinolinyl-Gruppen von A' ausgewählt sind aus der Gruppe bestehend aus Halogen, bis zu per-Halo und Wn, wobei n 0-3 ist und jedes W unabhängig ausgewählt ist aus der Gruppe bestehend aus C₁₋₁₀ Alkyl, C₁₋₁₀ Alkoxy, wenigstens einem fünfgliedrigen C₃₋₁₀ Cycloalkyl mit 0-3 Heteroatomen, C₂₋₁₀ Alkenyl, C₁₋₁₀ Alkenoyl, substituiertem C₁₋₁₀ Alkyl, substituiertem C₁₋₁₀ Alkoxy, wenigstens einem fünfgliedrigen substituierten C₃₋₁₀ Cycloalkyl mit 0-3 Heteroatomen, ausgewählt von N, S und O; -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, wobei jedes R⁷ und R^{7'} unabhängig ausgewählt ist aus Wasserstoff, C₁₋₁₀ Alkyl, C₁₋₁₀ Alkoxy, C₂₋₁₀ Alkenyl, C₁₋₁₀ Alkenoyl, bis zu per-Halo substituiertem C₁₋₁₀ Alkyl, bis zu per-Halo substituiertem C₁₋₁₀ Alkoxy, bis zu per-Halo substituiertem C₂₋₁₀ Alkenyl und bis zu per-Halo substituiertem C₁₋₁₀ Alkenoyl;
wobei B' substituiert ist, wobei die Substituenten ausgewählt sind aus der Gruppe bestehend aus Halogen, bis zu per-Halo und Jn, wobei n 0-3 ist und jedes J unabhängig ausgewählt ist aus der Gruppe bestehend aus -CN, -NO₂, -OR⁷, -SR⁷, -NR⁷R^{7'}, -NR⁷C(O)OR^{7'}, -NR⁷C(O)R^{7'}, wobei jedes R⁷ und R^{7'} unabhängig wie oben für W definiert ist, C₁₋₁₀ Alkyl, C₁₋₁₀ Alkoxy, wenigstens ein fünfgliedriges C₃₋₁₀ Cycloalkyl mit 0-3 Heteroatomen, C₂₋₁₀ Alkenyl, C₁₋₁₀ Alkenoyl, C₆₋₁₂ Aryl, wenigstens ein fünfgliedriges C₃₋₁₂ Hetaryl mit 1-3 Heteroatomen, ausgewählt aus N, S und O, C₇₋₂₄ Aralkyl, C₇₋₂₄ Alkaryl, substituiertes C₁₋₁₀ Alkyl, substituiertes C₁₋₁₀ Alkoxy, wenigstens ein fünfgliedriges substituiertes C₃₋₁₀ Cycloalkyl mit 0-3 Heteroatomen, ausgewählt aus N, S und O, substituiertes C₆₋C₁₄ Aryl, wenigstens einem fünfgliedrigem substituierten C₃₋₁₂ Hetaryl mit 1-3 Heteroatomen, ausgewählt aus N, S und O. substituiertes C₇₋₂₄ Alkaryl und substituiertes C₇-C₄ Aralkyl ist,
vorausgesetzt, dass L¹ nicht durch die Substituenten -C(O)R^{a}, -C(NR^{a})R^{b}, -C(O)NR^{a}R^{b} und -SO₂R^{a} ist, wobei R^{a} und R^{b} jeweils unabhängig Wasserstoff oder ein auf Kohlenstoff basierender Rest von bis zu 24 Kohlenstoffatomen ist, wahlweise enthaltend Heteroatome, ausgewählt aus N, S und O.

8. Verbindung nach Anspruch 7, wobei die Substituenten für B' ausgewählt sind aus der Gruppe bestehend aus -CN, Halogen, C₁₋₁₀ Alkyl, C₁₋₁₀ Alkoxy, -OH, bis zu per-Halo substituiertem C₁₋₁₀ Alkyl und bis zu per-Halo substituiertem C₁₋₁₀ Alkoxy.

9. Verbindung nach einem der Ansprüche 1 bis 8, wobei die substituierten Isoquinolinyle von A' 1-3 Substituenten aufweisen, die ausgewählt sind aus der Gruppe bestehend aus C₁₋₁₀ Alkyl, bis zu per-Halo substituiertem C₁₋₁₀ Alkyl, -CN, -OH, Halogen, C₁₋₁₀ Alkoxy, bis zu per-Halo substituiertem C₁₋₁₀ Alkoxy und wenigstens fünfgliedrigen C₃-C₁₀ heterozyklischen Resten, umfassend 1 bis 2 Heteroatome, ausgewählt aus der Gruppe bestehend aus Stickstoff, Sauerstoff und Schwefel.

10. Verbindung nach Anspruch 7 oder 8, wobei die substituierten Isoquinolinyle von A' 1-3 Substituenten aufweisen, die ausgewählt sind aus der Gruppe bestehend aus C₁₋₁₀ Alkyl, bis zu per-Halo substituiertem C₁₋₁₀ Alkyl, -CN, -OH, Halogen, C₁₋₁₀ Alkoxy, bis zu per-Halo substituiertem C₁₋₁₀ Alkoxy und wenigstens fünfgliedrigen C₃-C₁₀ heterozyklischen Resten, umfassend 1 bis 2 Heteroatome, ausgewählt aus der Gruppe bestehend aus Stickstoff, Sauerstoff und Schwefel.

11. Verbindung nach einem der Ansprüche 1 bis 10, wobei L und L' jeweils unabhängig Phenyl, substituiertes Phenyl, Pyridinyl, substituiertes Pyridinyl, Pyrimidinyl oder substituiertes Pyrimidinyl sind.

12. Verbindung nach einem der Ansprüche 1 bis 11, wobei L¹ 1 bis 3 mal durch einen oder mehreren der Substituenten substituiert ist, die ausgewählt sind aus der Gruppe bestehend aus C₁₋₁₀ Alkyl, bis zu per-Halo substituiertem C₁-C₁₀ Alkyl, bis zu per-Halo substituiertem C₁-C₁₀ Alkyl, -CN, -OH, Halogen, C₁-C₁₀ Alkoxy und bis zu per-Halo substituiertem C₁-C₁₀ Alkoxy.

13. Verbindung nach einem der Ansprüche 7, 8 und 10, wobei L¹ 1 bis 3 mal durch einen oder mehreren der Substituenten substituiert ist, die ausgewählt sind aus der Gruppe bestehend aus C₁₋₁₀ Alkyl, bis zu per-Halo substituiertem C₁-C₁₀ Alkyl, bis zu per-Halo substituiertem C₁-C₁₀ Alkyl, -CN, -OH, Halogen, C₁-C₁₀ Alkoxy und bis zu per-Halo substituiertem C₁-C₁₀ Alkoxy.

14. Verbindung nach Anspruch 1, wobei jeder Substituent J unabhängig ausgewählt ist aus der Gruppe bestehend aus C₁-C₁₀ Alkyl, bis zu per-Halo substituiertem C₁-C₁₀ Alkyl, -CN, -OH, Halogen, C₁-C₁₀ Alkoxy, bis zu per-Halo substituiertem C₁-C₁₀ Alkoxy, -CN, -CO₂ R⁷, -C(O)N R⁷ R^{7'}, -C(O)-R⁷, -NO₂, -O R7' -S R⁷, -N R⁷ R^{7'}, -N R⁷C(O)O R^{7'}, -N R⁷C(O) R^{7'}, wobei R⁷ und R^{7'}, jeweils unabhängig sind, wie für W in Anspruch 1 definiert,
M -O-, -S-, -N(R⁷)-, -(CH₂)ₘ-, -C(O)-, -CH(OH)-, -(CH₂)ₘO- und -O(CH₂)ₘ- ist, wobei m=1-3 und
L¹ Phenyl, Pyridinyl, Pyrimidinyl, substituiertes Phenyl, substituiertes Pyridinyl und substituiertes Pyrimidinyl ist mit Substituenten, die ausgewählt sind aus der Gruppe bestehend aus -CN; -OH; Halogen bis zu per-Halo; C₁-C₁₀ Alkoxy und halosubstituiertem C₁-C₁₀ Alkoxy, bis zu per-Halo.

15. Verbindung nach einem der Ansprüche 1 bis 14, die eine pharmazeutisch unbedenkliches Salz einer Verbindung der Formel II ist, ausgewählt aus der Gruppe bestehend aus
a) Hydroxidsalzen der organischen Säuren und anorganischen Säuren, ausgewählt aus der Gruppe bestehend aus Salzsäure, Bromwasserstoffsäure, Schwefelsäure, Phosphorsäure, Methanschwefelsäure, Trifluorschwefelsäure, Benzolschwefelsäure, p-Toluolsehwefelsäure (Tosylatsalz), 1-Napthalenschwefelsäure, 2-Napthalenschwefelsäure, Essigsäure, Schwefelsäure, 2-Napthalenschwefelsäure, Essigsäure, Trifluoressigsäure, Apfelsäure, Weinsäure, Zitronensäure, Milchsäure, Oxalsäure, Bernsteinsäure, Fumarsäure, Maleinsäure, Benzoesäure, Salicylsäure, Phenylessigsäure, und Mandelsäure sowie
b) Hydrogensalzen und anorganischen Basen, die Katione enthalten, die ausgewählt sind aus der Gruppe bestehend aus Alkalikationen, Erdalkalikationen, dem Ammoniumkation, aliphatischen substituierten Ammoniumkationen und aromatischen substituierten Ammoniumkationen.

16. Verbindung nach Anspruch 7, die ein pharmazeutisch unbedenkliches Salz von einer Verbindung der Formel II sind, ausgewählt aus der Gruppe bestehend aus
a) Hydroxidsalzen der organischen Säuren und anorganischen Säuren, ausgewählt aus der Gruppe bestehend aus Salzsäure, Bromwasserstoffsäure, Schwefelsäure, Phosphorsäure, Methanschwefelsäure, Trifluorschwefelsäure, Benzolschwefelsäure, p-Toluolschwefelsäure (Tosylatsalz), 1-Napthalenschwefelsäure, 2-Napthalenschwefelsäure, Essigsäure, Trifluoressigsäure, Apfelsäure, Weinsäure, Zitronensäure, Milchsäure, Oxalsäure, Bernsteinsäure, Fumarsäure, Maleinsäure, Benzoesäure, Salicylsäure, Phenylessigsäure, und Mandelsäure sowie
b) Hydrogensalzen und anorganischen Basen, die Katione enthalten, die ausgewählt sind aus der Gruppe bestehend aus Alkalikationen, Erdalkalikationen, dem Ammoniumkation, aliphatischen substituierten Ammoniumkationen und aromatischen substituierten Ammoniumkationen.

17. Pharmazeutische Zusammensetzung, umfassend eine Verbindung nach Anspruch 1 und einen physiologisch akzeptablen Träger.

18. Pharmazeutische Zusammensetzung, umfassend eine Verbindung nach Anspruch 7 und einen physiologisch akzeptablen Träger.

19. Verbindung, ausgewählt aus der Gruppe bestehend aus:
N-(3-Isoquinolinyl)-N'-(4-(4-Pyridinyloxy)Phenyl)Harnstoff;
N-(1-(4-Methylpiperazinyl)-3-Isoquinolinyl)-N'(4-(4-Pyridinyloxy)Phenyl)Harnstoff
oder einem pharmazeutisch unbedenklichen Salz davon.

20. Pharmazeutische Zusammensetzung, umfassend eine Verbindung nach Anspruch 19 und einen physiologisch akzeptablen Träger.

21. Verbindung nach Anspruch 1 einer der nachfolgenden Formeln 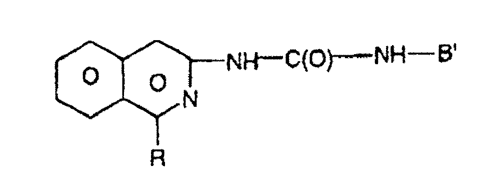 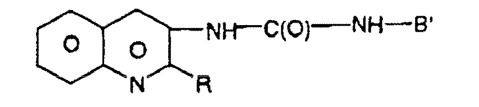 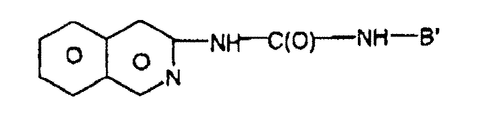 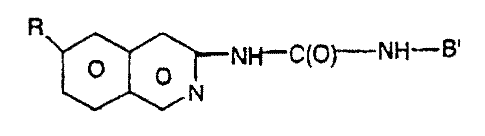 wobei B' wie in Anspruch definiert ist und R ausgewählt ist aus der Gruppe bestehend aus Halogen, C₁₋₁₀ Alkyl, C₁₋₁₀ Alkoxy, C₂₋₁₀ Alkenyl, C₁₋₁₀ Alkenoyl, -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, wobei jedes R⁷ und R^{7'} unabhängig ausgewählt ist aus Wasserstoff, C₁₋₁₀ Alkyl, C₁₋₁₀ Alkoxy, C₂₋₁₀ Alkenyl, C₁₋₁₀ Alkenoyl, bis zu per-Halo substituiertem C₁₋₁₀ Alkyl, bis zu per-Halo substituiertem C₁₋₁₀ Alkoxy, bis zu per-Halo substituiertem C₂₋₁₀ Alkenyl und bis zu per-Halo substituiertem C₁₋₁₀ Alkenoyl.

22. Verbindung nach Anspruch 1, wobei L ist:
(i) Phenyl, wahlweise substituiert durch 1-3 Substituenten, die unabhängig ausgewählt sind aus der Gruppe bestehend aus R⁷, OR⁷, NR⁷R^{7'}, C(O)R⁷, C(O)OR⁷, C(O)NR⁷R^{7'}, NR⁷C(O)R^{7'}, NR⁷C(O)OR^{7'}, Halogen, Cyan und Nitro oder
(ii) Pyridyl, wahlweise substituiert durch 1-3 Substituenten, die unabhängig ausgewählt sind aus der Gruppe bestehend aus R⁷, OR⁷, NR⁷R⁷', C(O)R⁷, C(O)OR⁷, C(O)NR⁷R^{7'}, NR⁷C(O)R^{7'}, NR⁷C(O)OR^{7'}, Halogen, Cyan und Nitro oder
(iii) Pyrimidinyl, wahlweise substituiert durch 1-3 Substituenten, die unabhängig ausgewählt sind aus der Gruppe bestehend aus R⁷, OR⁷, NR⁷R^{7'}, C(O)R⁷, C(O)OR⁷, C(O)NR⁷R^{7'}, NR⁷C(O)R^{7'}, NR⁷C(O)OR^{7'}, Halogen, Cyan und Nitro.

23. Verbindung nach Anspruch 1, wobei L¹ Phenyl, Pyridinyl oder Pyrimidinyl ist.

24. Verbindung nach Anspruch 1,
wobei L ist:
(i) Phenyl, wahlweise substituiert durch 1-3 Substituenten, die unabhängig ausgewählt sind aus der Gruppe bestehend aus C₁-C₅ linearem oder verzweigtem Alkyl, C₁-C₅ linearem oder verzweigtem Haloalkyl, C₁-C₃ Alkoxy, Hydroxy, Amino, C₁-C₃ Alkylamino, C₁-C₆ Dialkylamino, Halogen, Cyano und Nitro oder
(ii) Pyridyl, wahlweise substituiert durch 1-3 Substituenten, die unabhängig ausgewählt sind aus der Gruppe bestehend aus C₁-C₅ linearem oder verzweigtem Alkyl, C₁-C₅ linearem oder verzweigtem Haloalkyl, C₁-C₃ Alkoxy, Hydroxy, Amino, C₁-C₃ Alkylamino, C₁-C₆ Dialkylamino, Halogen, Cyano und Nitro und
L¹ einen zyklischen Rest umfasst, der ausgewählt ist aus der Gruppe bestehend aus:
(ii) Naphtyl, wahlweise substituiert durch 1-3 Substituenten, die unabhängig ausgewählt sind aus der Gruppe bestehend aus R⁷, OR⁷, NR⁷R^{7'}, NR⁷C(O)R^{7'}, NR⁷C(O)OR^{7'}, Halogen, Cyan und Nitro;
(iii) Pyridyl, wahlweise substituiert durch 1-3 Substituenten, die unabhängig ausgewählt sind aus der Gruppe bestehend aus R⁷, OR⁷, NR⁷R^{7'}, NR⁷C(O)R^{7'}, NR⁷C(O)OR^{7'} , Halogen, Cyan und Nitro;
(iv) Pyrimidinyl, wahlweise substituiert durch 1-3 Substituenten, die unabhängig ausgewählt sind aus der Gruppe bestehend aus R⁷, OR⁷, NR⁷R^{7'}, NR⁷C(O)R^{7'}, NR⁷C(O)OR^{7'}, Halogen, Cyan und Nitro;
(v) Quinolinyl, wahlweise substituiert durch 1-3 Substituenten, die unabhängig ausgewählt sind aus der Gruppe bestehend aus R⁷, OR⁷, NR⁷R^{7'}, NR⁷C(O)R^{7'}, NR⁷C(O)OR^{7'}, Halogen, Cyan und Nitro;
(vi) Isoquinolinyl, wahlweise substituiert durch 1-3 Substituenten, die unabhängig ausgewählt sind aus der Gruppe bestehend aus R⁷, OR⁷, NR⁷R^{7'}, NR⁷C(O)R^{7'}, NR⁷C(O)OR^{7'}, Halogen, Cyan und Nitro
und wobei jedes R⁷ und R^{7'} unabhängig
(a) Wasserstoff,
(b) C₁-C₆ lineares oder verzweigtes Alkyl, bis zu per-Halo substituiertes C₁-C₅ lineares oder verzweigtes Alkyl, C₁-C₃ Alkoxy und Hydroxy;
(c) C₁-C₆ Alkoxy, wahlweise substituiert durch 1-3 Substituenten, die unabhängig ausgewählt sind aus der Gruppe bestehend aus C₁-C₅ linearem oder verzweigtem Alkyl, bis zu per-Halo substituiertem C₁-C₅ linearem oder verzweigtem Alkyl, C₁-C₃ Alkoxy, Hydroxy und Halogen;
(d) Phenyl, wahlweise substituiert durch 1-3 Substituenten, die unabhängig ausgewählt sind aus der Gruppe bestehend aus C₁-C₅ linearem oder verzweigtem Alkyl, bis zu per-Halo substituiertem C₁-C₅ linearem oder verzweigtem Alkyl, C₁-C₃ Alkoxy, Hydroxy und Halogen;
(e) 5- bis 6-gliedriges monozyklisches Heteroaryl mit 1-4 Heteroatomen, ausgewählt aus der Gruppe bestehend aus O, N und S oder 8- bis 10-gliedriges bizyklisches Heteroaryl mit 1-6 Heteroatomen, ausgewählt aus der Gruppe bestehend aus O, N und S, wahlweise substituiert durch 1-3 Substituenten, die unabhängig ausgewählt sind aus der Gruppe bestehend aus C₁-C₅ linearem oder verzweigtem Alkyl, bis zu per-Halo substituiertem C₁-C₅ linearem oder verzweigtem Alkyl, C₁-C₃ Alkoxy, Hydroxy und Halogen,
(f) C₁-C₃ Alkyl-Phenyl, wahlweise substituiert durch 1-3 Substituenten, die unabhängig ausgewählt sind aus der Gruppe bestehend aus C₁-C₅ linearem oder verzweigtem Alkyl, bis zu per-Halo substituiertem C₁-C₅ linearem oder verzweigtem Alkyl, C₁-C₃ Alkoxy, Hydroxy und Halogen und
(g) bis zu per-Halo substituiertes C₁-C₅ lineares, verzweigtes oder zyklisches Alkyl ist und wo nicht per-Halo substituiert, wahlweise substituiert durch 1-3 Substituenten, die unabhängig ausgewählt sind aus der Gruppe bestehend aus C₁-C₅ linearem oder verzweigtem Alkyl, bis zu per-Halo substituiertem C₁-C₅ linearem oder verzweigtem Alkyl, C₁-C₃ Alkoxy, Hydroxy, ist.

25. Verbindung nach Anspruch 1, wobei A', L und L¹ einer der nachfolgenden Kombinationen folgen:
A' = Isoquinolinyl, L = Phenyl und L¹ ist Phenyl, Pyridinyl, Quinolinyl oder Isoquinolinyl.
A' = Isoquinolinyl, L = Pyridinyl und L¹ ist Phenyl, Pyridinyl, Quinolinyl oder Isoquinolinyl,
A' = Quinolinyl, L = Phenyl und L¹ ist Phenyl, Pyridinyl, Quinolinyl oder Isoquinolinyl,
A' = Quinolinyl, L = Pyridinyl und L¹ ist Phenyl, Pyridinyl, Quinolinyl oder Isoquinolinyl,
A' = substituiertes Quinolinyl, L = Phenyl und L¹ ist Phenyl, Pyridinyl, Quinolinyl oder Isoquinolinyl oder
A' = substituiertes Quinolinyl, L = Pyridinyl und L¹ ist Phenyl, Pyridinyl, Quinolinyl oder Isoquinolinyl.

26. Verbindung nach Anspruch 1, wobei L¹ Pyridyl ist.

27. Verwendung einer Verbindung nach einem der Ansprüche 1 bis 26 zur Herstellung eines Medikaments zur Behandlung einer Krankheit, bei der die Hemmung des Raf-Kinasewegs erforderlich ist.

28. Verwendung einer Verbindung nach einem der Ansprüche 1 bis 26 zur Herstellung eines Medikaments zur Behandlung eines Tumors und/oder eines durch Raf-Kinase vermittelten krebsartigen Zellwachstums.

29. Verwendung nach Anspruch 27 oder 28, wobei es sich bei der genannten Krankheit um Mäusekrebs, solide Krebsarten, wie z.B. Karzinomen, beispielsweise der Lunge, der Bauchspeicheldrüse, der Schilddrüse, der Blase oder des Darms, um Knochenmarkerkrankungen, wie z.B. Knochenmarkleukämie oder um Drüsengeschwülste, wie z.B. villöse Darmdrüsengeschwülste, handelt.

## Revendications

1. Composé de formule suivante :
A'-D-B' (II)
ou sel pharmaceutiquement acceptable de celui-ci, où
D est -NH-C(O)-NH-,
A' est un groupe isoquinolinyle substitué ou un groupe isoquinolinyle non substitué ou un groupe quinolinyle non substitué,
B' est une structure cyclique pontée substituée ou non substituée de jusqu'à 30 atomes de carbone de formule -L-(ML¹)_{q} où L comprend un groupement cyclique ayant au moins cinq membres et est lié directement à D, L' comprend un groupe cyclique ayant au moins cinq membres, M est choisi dans le groupe consistant en -O-, -S-, -N(R⁷)-, -(CH₂)ₘ, -C(O)-, -CH(OH)-, -(CH₂)ₘO-, -(CH₂)ₘS-, -(CH₂)ₘN(R⁷)-, -O(CH₂)ₘ- ; -CHX^{a}, -CX^{a}₂-, -S-(CH₂)ₘ- et -N(R⁷)(CH₂)ₘ-, où m = 1-3, X^{a} est halogène, et R⁷ est comme défini ci-dessous, q est un entier de 1-3, et chaque structure cyclique de L et L¹ contient 0-4 membres du groupe consistant en l'azote, l'oxygène et le soufre, soumis à la condition que B' n'est pas 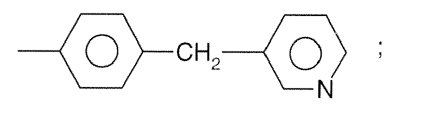 ou 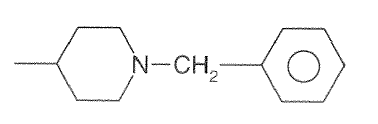
où les substituants pour les groupes isoquinolinyle substitués de A' sont choisis dans le groupe consistant en halogène, jusqu'à per-halogéno, et Wn, où n est 0-3 et chaque W est choisi indépendamment dans le groupe consistant en C₁₋₁₀ alkyle, C₁₋₁₀ alcoxy, au moins un C₃₋₁₀ cycloalkyle à cinq membres ayant 0-3 hétéroatomes, C₂₋₁₀ alcényle, C₁₋₁₀ alcénoyle, C₁₋₁₀ alkyle substitué, C₁₋₁₀ alcoxy substitué, au moins un C₃₋₁₀ cycloalkyle substitué à cinq membres ayant 0-3 hétéroatomes choisis parmi N, S et O ; -CN, C₆-C₁₄ aryle jusqu'à per-halogéno-substitué, C₇-C₂₄ alkaryle jusqu'à per-halogéno substitué, C₇-C₂₄ aralkyle jusqu'à per-halogéno substitué, C₃-C₁₂ hétéroaryle jusqu'à per-halogéno substitué ayant au moins cinq membres et 1-3 hétéroatomes choisis parmi O, N et S, C₄-C₂₄ alkhétéroaryle jusqu'à per-halogéno substitué ayant au moins cinq membres et 1-3 hétéroatomes choisis parmi 0, N et S, C₆-C₁₄ aryle, C₁-C₂₄ alkaryle, C₁-C₂₄ aralkyle, C₃-C₁₂ hétéroaryle ayant au moins cinq membres cycliques et 1-3 hétéroatomes choisis parmi O, N et S, C₄-C₂₄ alkhétéroaryle ayant au moins cinq membres cycliques et 1-3 hétéroatomes choisis parmi O, N et S ; -CO₂R⁷, -C(O)NR⁷R⁷', -C(O)-R⁷, -NO₂, -OR⁷, -SR⁷, -NR⁷R⁷', -NR⁷C(O)OR⁷', -NR⁷C(O)R⁷', avec chaque R⁷ et R⁷' choisi indépendamment parmi l'hydrogène, C₁₋₁₀ alkyle, C₁₋₁₀ alcoxy, C₂₋₁₀ alcényle, C₁₋₁₀ alcénoyle, C₁₋₁₀ alkyle jusqu'à per-halogéno substitué, C₁₋₁₀ alcoxy jusqu'à per-halogéno substitué, C₂₋₁₀ alcényle jusqu'à per-halogéno substitué et C₁₋₁₀ alcénoyle jusqu'à per-halogéno substitué ;
quand B' est substitué, les substituants sont choisis dans le groupe consistant en halogène, jusqu'à per-halogéno, et Jn, où n est 0-3 et chaque J est choisi indépendamment dans le groupe consistant en -CN, -CO₂R⁷, -C(O)NR⁷R⁷', -C(O)-R⁷, -NO₂, -OR⁷, -SR⁷, -NR⁷R⁷', -NR⁷C(O)OR^{7'}, -NR⁷C(O)R⁷', avec chaque R⁷ et R^{7'} indépendamment tel que défini ci-dessus pour W, C₁₋₁₀ alkyle, C₁₋₁₀ alcoxy, au moins un C₃-C₁₀ cycloalkyle à cinq membres ayant 0-3 hétéroatomes, C₂-C₁₀ alcényle, C₁-C₁₀ alcénoyle, C₆₋₁₂ aryle, au moins un C₃₋₁₂ hétéroaryle à cinq membres ayant 1-3 hétéroatomes choisis parmi N, S et O, C₇₋₂₄ aralkyle, C₇₋₂₄ alkaryle, C₁₋₁₀ alkyle substitué, C₁₋₁₀ alcoxy substitué, au moins un C₃₋₁₀ cycloalkyle substitue à cinq membres ayant 0-3 hétéroatomes choisis parmi N, S et O, C₆-C₁₄ aryle substitué, au moins un C₃₋₁₂ hétéroaryle substitué à J membres ayant 1-3 hétéroatomes choisis parmi N, S et O, C₇₋₂₄ alkaryle substitué et C₇-C₂₄ aralkyle substitué,
soumis à la condition que quand B' est -L(ML¹)_{q}, L¹ n'est pas substitué par les substituants -C(O)R^{a}, -C(NR^{a})R^{b}, -C(O)NR^{a}R^{b} et -SO₂R^{a} où chaque R^{a} et R^{b} sont indépendamment hydrogène ou un groupement à base de carbone de jusqu'à 24 atomes de carbone, contenant éventuellement des hétéroatomes choisis parmi N, S et O,
quand J est un groupe substitué, il est substitué par halogène, jusqu'à per-halogéno, ou par un ou plusieurs substituants choisis indépendamment dans le groupe consistant en -CN, -CO₂R⁷, -OR⁷, -SR⁷, -NR⁷R^{7'}, -NO₂, -NR⁷C(O)R^{7'} et -NR⁷C(O)OR^{7'} ; avec chaque R⁷ et R^{7'} indépendamment comme défini ci-dessus pour W.

2. Composé selon la revendication 1 où :
R^{a} et R^{b} sont, indépendamment, C₁₋₁₀ alkyle, C₁₋₁₀ alcoxy, C₃₋₁₀ cycloalkyle ayant 0-3 hétéroatomes, C₂₋₁₀ alcényle, C₁₋₁₀ alcénoyle, C₆₋₁₂ aryle, C₃₋₁₂ hétéroaryle ayant 1-3 hétéroatomes choisis parmi N, S et O, C₇₋₂₄ aralkyle, C₇₋₂₄ alkaryle, C₁₋₁₀ alkyle substitué, C₁₋₁₀ alcoxy substitué, C₃₋₁₀ cycloalkyle substitué ayant 0-3 hétéroatomes choisis parmi N, S et O, C₆-C₁₄ aryle substitué, C₃₋₁₂ hétéroaryle substitué ayant 1-3 hétéroatomes choisis parmi N, S et O, C₇₋₂₄ alkaryle substitué ou C₇-C₂₄ aralkyle substitué, quand R^{a} est un groupe substitué, il est substitué par halogène jusqu'à per-halogéno.

3. composé selon la revendication 1 ou 2 où L dans la formule -L-(ML¹)_{q} pour B' est un groupe aryle cyclique à 6 membres substitué, un groupement hétéroaryle cyclique à 5-6 membres substitué, un groupement aryle cyclique à 6 membres non substitué ou un groupement hétéroaryle cyclique à 5-6 membres non substitué, et L¹ dans la formule -L-(ML¹)_{q} de B' est un groupement aryle substitué ayant au moins 6 membres cycliques, un groupement aryle non substitué ayant au moins 6 membres cycliques, un groupement hétéroaryle substitué ayant au moins 5 membres cycliques ou un groupement hétéroaryle non substitué ayant au moins 5 membres cycliques, lesdits groupements hétéroaryle ayant 1 à 4 membres choisis dans le groupe d'hétéroatomes consistant en l'azote, l'oxygène et le soufre, le complément du groupement hétéroaryle étant du carbone.

4. Composé selon l'une quelconque des revendications 1 à 3 où L et L' sont choisis chacun indépendamment dans le groupe consistant en thiophène, phényle, phényle substitué, pyridinyle, pyridinyle substitué, pyrimidinyle, pyrimidinyle substitué, naphtyle, naphtyle substitué, quinolinyle, quinolinyle substitué, isoquinodiyle et isoquinodiyle substitué.

5. Composé selon l'une quelconque des revendications 1 à 4, où B' est un groupe substitué, il est substitué par -CN, halogène, C₁₋₁₀ alkyle, C₁₋₁₀ alcoxy, -OH, C₁₋₁₀ alkyle jusqu'à per-halogéno substitué, C₁₋₁₀ alcoxy jusqu'à per-halogéno substitué, -O(R⁷), -SR⁷, -NR⁷R^{7'} ou -NO₂, où chaque R⁷ et R^{7'} sont choisis indépendamment parmi l'hydrogène, C₁₋₁₀ alkyle, C₁₋₁₀ alcoxy, C₂₋₁₀ alcényle, C₁₋₁₀ alcénoyle, C₁₋₁₀ alkyle jusqu'à per-halogéno substitué, C₁₋₁₀ alcoxy jusqu'à per-halogéno substitué, C₂₋₁₀ alcényle jusqu'à per-halogéno substitué et C₁₋₁₀ alcénoyle jusqu'à per-halogéno substitué.

6. Composé selon l'une quelconque des revendications 1 à 5, où M dans la formule -L-(ML¹) pour B' est -O-, -CH₂-, -S-, -NH-, -C(O)-, -O-CH₂ ou -CH₂-O-.

7. Composé de formule A'-D-B', ou sel pharmaceutiquement acceptable de celui-ci, où D est -NH-C(O)-NH-
A' est un groupe isoquinolinyle substitué ou un groupe isoquinolinyle non substitué ou un groupe quinolinyle non substitué,
B' est de formule -L-(ML¹)q, où L est phényle ou phényle substitué et L¹ est phényle, phényle substitué, pyridinyle ou pyridinyle substitué, q est un entier de 1-2 et M est choisi dans le groupe consistant en -O-, -S-, -N(R⁷)-, -(CH₂)ₘ, -C(O)-, -CH(OH)-, -(CH₂)ₘO-, -(CH₂)ₘS-, -(CH₂)ₘN(R⁷)-, -O(CH₂)ₘ-; -CHX^{a}, -CX^{a}₂-, -S-(CH₂)ₘ- et N(R⁷)(CH₂)ₘ-, où m = 1-3, X^{a} est halogène, et R⁷ est tel que défini ci-dessous, soumis à la condition que B' n'est pas 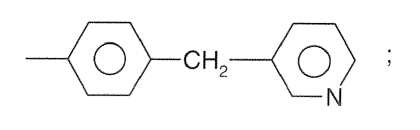
où les substituants pour les groupes isoquinolinyle substitués de A' sont choisis dans le groupe consistant en halogène, jusqu'à per-halogéno, et Wn, où n est 0-3 et chaque W est choisi indépendamment dans le groupe consistant en C₁₋₁₀ alkyle, C₁₋₁₀ alcoxy, au moins un C₃₋₁₀ cycloalkyle à cinq membres ayant 0-3 hétéroatomes, C₂₋₁₀ alcényle, C₁₋₁₀ alcénoyle, C₁₋₁₀ alkyle substitué, C₁₋₁₀ alcoxy substitué, au moins un C₃₋₁₀ cycloalkyle substitué à cinq membres ayant 0-3 hétéroatomes choisis parmi N, S et O; -CN, -CO₂R⁷, -C(O)NR⁷R⁷', -C(O)-R⁷, -NO₂, -OR⁷, -SR⁷, -NR⁷R⁷', -NR⁷C(O)OR⁷', -NR⁷C(O)R⁷', avec chaque R⁷ et R^{7'} choisi indépendamment parmi hydrogène, C₁₋₁₀ alkyle, C₁₋₁₀ alcoxy, C₂₋₁₀ alcényle, C₁₋₁₀ alcénoyle, C₁₋₁₀ alkyle jusqu'à per-halogéno substitué, C₁-C₁₀ alcoxy jusqu'à per-halogéno substitué, C₂₋₁₀ alcényle jusqu'à per-halogéno substitué et C₁₋₁₀ alcénoyle jusqu'à per-halogéno substitué;
où B' est substitué, les substituants sont choisis dans le groupe consistant en halogène, jusqu'à per-halogéno, et Jn, où n est 0-3 et chaque J est choisi indépendamment dans le groupe consistant en -CN, -NO₂, -OR⁷, -SR⁷, -NR⁷R^{7'}, -NR⁷C(O)OR^{7'}, -NR⁷C(O)R^{7'} ; avec chaque R⁷ et R^{7'} indépendamment comme défini ci-dessus pour W, C₁₋₁₀ alkyle, C₁₋₁₀ alcoxy, au moins un C₃₋₁₀ cycloalkyle à 5 membres ayant 0-3 hétéroatomes, C₂₋₂₀ alcényle, C₁₋₁₀ alcénoyle, C₆₋₁₂ aryle, au moins un C₃₋₁₂ hétéroaryle à cinq membres ayant 1-3 hétéroatomes choisis parmi N, S et 0, C₇₋₂₄ aralkyle, C₇₋₂₄ alkaryle, C₁₋₁₀ alkyle substitué, C₁₋₁₀ alcoxy substitué, au moins un C₃₋₁₀ cycloalkyle substitué à 5 membres ayant 0-3 hétéroatomes choisis parmi N, S et O, C₆-C₁₄ aryle substitué, au moins un C₃₋₁₂ hétéroaryle substitué à 5 membres ayant 1-3 hétéroatomes choisis parmi N, S et O, C₇₋₂₄ alkaryle substitué et C₇-C₂₄ aralkyle substitué, soumis à la condition que L¹ n'est pas substitué par les substituants -C(O)R^{a}, -C(NR^{a})R^{b}, -C(O)NR^{a}R^{b} et -SO₂R^{a} où R^{a} et R^{b} sont indépendamment hydrogène ou un groupement à base de carbone de jusqu'à 24 atomes de carbone, contenant éventuellement des hétéroatomes choisis parmi N, S et O.

8. Composé selon la revendication 7 où les substituants pour B' sont choisis dans le groupe consistant en -CN, halogène, C₁₋₁₀ alkyle, C₁₋₁₀ alcoxy, -OH, C₁₋₁₀ alkyle jusqu'à per-halogéno substitué, et C₁₋₁₀ alcoxy jusqu'à per-halogéno substitué.

9. Composé selon l'une quelconque des revendications 1 à 8 où les isoquinolinyles substitués de A' ont 1-3 substituants choisis dans le groupe consistant en C₁₋₁₀ alkyle, C₁₋₁₀ alkyle jusqu'à per-halogéno substitué, -CN, -OH, halogène, C₁₋₁₀ alcoxy, C₁₋₁₀ alcoxy jusqu'à per-halogéno substitué et au moins un groupement C₃-C₁₀ hétérocyclique à 5 membres comprenant 1 à 2 hétéroatomes choisis dans le groupe consistant en l'azote, l'oxygène et le soufre.

10. Composé selon la revendication 7 ou 8, où les isoquinolinyles substitués de A' ont 1-3 substituants choisis dans le groupe consistant en C₁₋₁₀ alkyle, C₁₋₁₀ alkyle jusqu'à per-halogéno substitué, -CN, -OH, halogène, C₁₋₁₀ alcoxy, C₁₋₁₀ alcoxy jusqu'à per-halogéno substitué et au moins un groupement C₃₋₁₀ hétérocyclique à 5 membres comprenant 1 à 2 hétéroatomes choisis dans le groupe consistant en l'azote, l'oxygène et le soufre.

11. Composé selon l'une quelconque des revendications 1 à 10 où L et L¹ sont indépendamment phényle, phényle substitué, pyridinyle, pyridinyle substitué, pyrimidinyle ou pyrimidinyle substitué.

12. Composé selon l'une quelconque des revendications 1 à 11 où L¹ est substitué 1 à 3 fois par un ou plusieurs substituants choisis dans le groupe consistant en C₁-C₁₀ alkyle, C₁-C₁₀ alkyle jusqu'à per-halogéno substitué, -CN, -OH, halogène, C₁-C₁₀ alcoxy et C₁-C₁₀ alcoxy jusqu'à per-halogéno substitué.

13. Composé selon l'une quelconque des revendications 7, 8 et 10, où L¹ est substitué une à trois fois par un ou plusieurs substituants choisis dans le groupe consistant en C₁-C₁₀ alkyle, C₁-C₁₀ alkyle jusqu'à per-halogéno substitué, -CN, -OH, halogène, C₁-C₁₀ alcoxy et C₁-C₁₀ alcoxy jusqu'à per-halogéno substitué.

14. Composé selon la revendication 1 où chaque substituant J est choisi indépendamment dans le groupe consistant en C₁-C₁₀ alkyle, C₁-C₁₀ alkyle jusqu'à per-halogéno substitué, -CN, -OH, halogène, C₁-C₁₀ alcoxy, C₁-C₁₀ alcoxy jusqu'à per-halogéno substitué, -CN, -CO₂R⁷, -C(O)NR⁷R⁷', -C(O)-R⁷, -NO₂, -OR⁷, -SR⁷, -NR⁷R⁷', -NR⁷C(O)OR⁷' et -NR⁷C(O)R⁷', où R⁷ et R^{7'} sont chacun indépendamment comme défini pour W dans la revendication 1,
M est -O-, -S-, -N(R⁷)-, -(CH₂)ₘ-, -C(O)-, -CH(OH)-, -(CH₂)ₘO- et -O(CH₂)ₘ-, où m = 1-3 ; et
L¹ est phényle, pyridinyle, pyrimidinyle, phényle substitué, pyridinyle substitué et pyrimidinyle substitué, avec des substituants choisis dans le groupe consistant en -CN ; -OH ; halogène jusqu'à per-halogéno ; C₁-C₁₀ alcoxy et C₁-C₁₀ alcoxy halogéno substitué, jusqu'à per-halogéno.

15. Composé selon l'une quelconque des revendications 1 à 14 qui est un sel pharmaceutiquement acceptable d'un composé de formule II choisi dans le groupe consistant en
a) les sels basiques d'acides organiques et d'acides inorganiques choisis dans le groupe consistant en l'acide chlorhydrique, l'acide bromhydrique, l'acide sulfurique, l'acide phosphorique, l'acide méthanesulfonique, l'acide trifluorosulfonique, l'acide benzènesulfonique, l'acide p-toluènesulfonique (sel tosylate), l'acide 1-naphtalènesulfonique, l'acide 2-naphtalènesulfonique, l'acide acétique, l'acide sulfonique, l'acide 2-naphtalènesulfonique, l'acide acétique, l'acide trifluoroacétique, l'acide malique, l'acide tartrique, l'acide citrique, l'acide lactique, l'acide oxalique, l'acide succinique, l'acide fumarique, l'acide maléique, l'acide benzoïque, l'acide salicylique, l'acide phénylacétique et l'acide mandélique ; et
b) les sels d'acides de bases organiques et inorganiques contenant des cations choisis dans le groupe consistant en les cations alcalins, les cations alcalino-terreux, le cation ammonium, les cations ammonium à substitution aliphatique et les cations ammonium à substitution aromatique.

16. Composé selon la revendication 7 qui est un sel pharmaceutiquement acceptable d'un composé de formule II choisi dans le groupe consistant en
a) les sels basiques d'acides organiques et d'acides inorganiques choisis dans le groupe consistant en l'acide chlorhydrique, l'acide bromhydrique, l'acide sulfurique, l'acide phosphorique, l'acide méthanesulfonique, l'acide trifluoro-sulfonique, l'acide benzènesulfonique, l'acide p-toluène-sulfonique (sel tosylate), l'acide 1-naphtalènesulfonique, l'acide 2-naphtalènesulfonique, l'acide acétique, l'acide trifluoroacétique, l'acide malique, l'acide tartrique, l'acide citrique, l'acide lactique, l'acide oxalique, l'acide succinique, l'acide fumarique, l'acide maléique, l'acide benzoïque, l'acide salicylique, l'acide phénylacétique et l'acide mandélique ; et
b) les sels d'acides de bases organiques et inorganiques contenant des cations choisis dans le groupe consistant en les cations alcalins, les cations alcalino-terreux, le cation ammonium, les cations ammonium à substitution aliphatique et les cations ammonium à substitution aromatique.

17. Composition pharmaceutique comprenant un composé selon la revendication 1 et un support physiologiquement acceptable.

18. Composition pharmaceutique comprenant un composé selon la revendication 7 et un support physiologiquement acceptable.

19. Composé choisi dans le groupe consistant en :
N-(3-isoquinolinyl)-N'-(4-(4-pyridinyloxy)phényl)urée ;
N-(1-(4-méthylpipérazinyl)-3-isoquinolinyl)-N'-(4-(4-pyridinyloxy)phényl)-urée ;
ou sel pharmaceutiquement acceptable de celui-ci.

20. Composition pharmaceutique comprenant un composé selon la revendication 19 et un support physiologiquement acceptable.

21. Composé selon la revendication 1 de l'une des formules suivantes 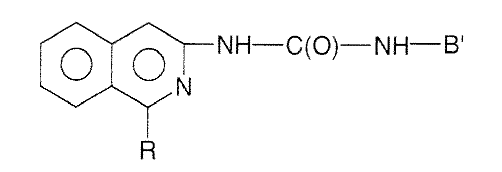 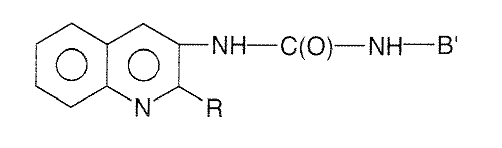 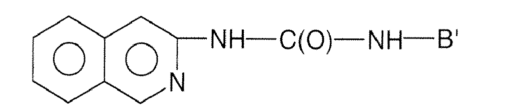 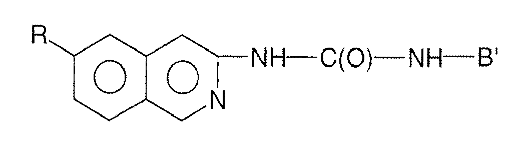 où B' est tel que défini dans la revendication 1 et R est choisi dans le groupe consistant en halogène, C₁₋₁₀ alkyle, C₁₋₁₀ alcoxy, C₂₋₁₀ alcényle, C₁₋₁₀ alcénoyle, -CN, -CO₂R⁷, -C(O)NR⁷R⁷', -C(O)-R⁷, -NO₂, -OR⁷, -SR⁷, -NR⁷R⁷', -NR⁷C(O)OR⁷', -NR⁷C(O)R⁷', avec chaque R⁷ et R⁷' sont choisis indépendamment parmi hydrogène, C₁₋₁₀ alkyle, C₁₋₁₀ alcoxy, C₂₋₁₀ alcényle, C₁₋₁₀ alcénoyle, C₁₋₁₀ alkyle jusqu'à per-halogéno substitué, C₁₋₁₀ alcoxy jusqu'à per-halogéno substitué, C₂₋₁₀ alcényle jusqu'à per-halogéno substitué et C₁₋₁₀ alcénoyle jusqu'à per-halogéno substitué.

22. Composé selon la revendication 1 où L est :
(i) phényle, éventuellement substitué avec 1-3 substituants choisis indépendamment dans le groupe consistant en R⁷, OR⁷, NR⁷R⁷', C(O)R⁷, C(O)OR⁷, C(O)NR⁷R^{7'}, NR⁷C(O)R^{7'}, NR⁷C(O)OR^{7'}, halogène, cyano et nitro ; ou
(ii) pyridyle, éventuellement substitué avec 1-3 substituants choisis indépendamment dans le groupe consistant en R⁷, OR⁷, NR⁷R^{7'}, C(O)R⁷, C(O)OR⁷, C(O)NR⁷R^{7'}, NR⁷C(O)R^{7'}, NR⁷C(O)OR^{7'}, halogène, cyano et nitro ; ou
(iii) pyrimidinyle, éventuellement substitué avec 1-3 substituants choisis indépendamment dans le groupe consistant en R⁷, OR⁷, NR⁷R⁷', C(O)R⁷, C(O)OR⁷, C(O)NR⁷R^{7'}, NR⁷C(O)R^{7'}, NR⁷C(O)OR^{7'}, halogène, cyano et nitro.

23. Composé selon la revendication 1 où L¹ est phényle, pyridinyle ou pyrimidinyle.

24. Composé selon la revendication 1,
où L est :
(i) phényle, éventuellement substitué avec 1-3 substituants choisis indépendamment dans le groupe consistant en C₁-C₅ alkyle linéaire ou ramifié, C₁-C₅ halogénoalkyle linéaire ou ramifié, C₁-C₃ alcoxy, hydroxy, amino, C₁-C₃ alkylamino, C₁-C₆ dialkylamino, halogène, cyano et nitro ; ou
(ii) pyridyle, éventuellement substitué avec 1-3 substituants choisis indépendamment dans le groupe consistant en C₁-C₅ alkyle linéaire ou ramifié, C₁-C₅ halogénoalkyle linéaire ou ramifié, C₁-C₃ alcoxy, hydroxy, amino, C₁-C₃ alkylamino, C₁-C₆ dialkylamino, halogène, cyano et nitro ; et
L¹ comprend un groupement cyclique choisi dans le groupe consistant en :
(ii) naphtyle, éventuellement substitué avec 1-3 substituants choisis indépendamment dans le groupe consistant en R⁷, OR⁷, NR⁷R⁷', NR⁷C(O)R^{7'}, NR⁷C(O)OR^{7'}, halogène, cyano et nitro ;
(iii) pyridyle, éventuellement substitué avec 1-3 substituants choisis indépendamment dans le groupe consistant en R⁷, OR⁷, NR⁷R⁷', NR⁷C(O)R^{7'}, NR⁷C(O)OR^{7'}, halogène, cyano et nitro ;
(iv) pyrimidinyle, éventuellement substitué avec 1-3 substituants choisis indépendamment dans le groupe consistant en R⁷, OR⁷, NR⁷R⁷', NR⁷C(O)R^{7'}, NR⁷C(O)OR^{7'}, halogène, cyano et nitro ;
(v) quinolinyle, éventuellement substitué avec 1-3 substituants choisis indépendamment dans le groupe consistant en R⁷, OR⁷, NR⁷R⁷', NR⁷C(O)R^{7'}, NR⁷C(O)OR^{7'}, halogène, cyano et nitro ;
(vi) isoquinolinyle, éventuellement substitué avec 1-3 substituants choisis indépendamment dans le groupe consistant en R⁷, OR⁷, NR⁷R⁷', NR⁷C(O)R^{7'}, NR⁷C(O)OR^{7'}, halogène, cyano et nitro ;
et chaque R⁷ et R^{7'} est indépendamment
(a) l'hydrogène,
(b) C₁-C₆ alkyle linéaire, ramifié ou cyclique, éventuellement substitué avec 1-3 substituants choisis indépendamment dans le groupe consistant en C₁-C₅ alkyle linéaire ou ramifié, C₁-C₅ alkyle linéaire ou ramifié jusqu'à per-halogéno substitué, C₁-C₃ alcoxy et hydroxy ;
(c) C₁-C₆ alcoxy, éventuellement substitué avec 1-3 substituants choisis indépendamment dans le groupe consistant en C₁-C₅ alkyle linéaire ou ramifié, C₁-C₅ alkyle linéaire ou ramifié jusqu'à per-halogéno substitué, C₁-C₃ alcoxy, hydroxy et halogène ;
(d) phényle, éventuellement substitué avec 1-3 substituants choisis indépendamment dans le groupe consistant en C₁-C₅ alkyle linéaire ou ramifié, C₁-C₅ alkyle linéaire ou ramifié jusqu'à per-halogéno substitué, C₁-C₃ alcoxy, hydroxy et halogène,
(e) hétéroaryle monocyclique à 5-6 membres ayant 1-4 hétéroatomes choisis dans le groupe consistant en O, N et S ou hétéroaryle bicyclique à 8-10 membres ayant 1-6 hétéroatomes choisis dans le groupe consistant en 0, N et S, éventuellement substitué avec 1-3 substituants choisis indépendamment dans le groupe consistant en C₁-C₅ alkyle linéaire ou ramifié, C₁-C₅ alkyle linéaire ou ramifié jusqu'à per-halogéno substitué, C₁-C₃ alcoxy, hydroxy et halogène,
(f) C₁-C₃ alkyle-phényle, éventuellement substitué avec 1-3 substituants choisis indépendamment dans le groupe consistant en C₁-C₅ alkyle linéaire ou ramifié, C₁-C₅ alkyle linéaire ou ramifié jusqu'à per-halogéno substitué, C₁-C₃ alcoxy, hydroxy et halogène; et
(g) C₁-C₅ alkyle linéaire, ramifié ou cyclique jusqu'à per-halogéno substitué et quand il n'est pas per-halogéno substitué, éventuellement substitué avec 1-3 substituants choisis indépendamment dans le groupe consistant en C₁-C₅ alkyle linéaire ou ramifié, C₁-C₅ alkyle linéaire ou ramifié jusqu'à per-halogéno substitué, C₁-C₃ alcoxy et hydroxy.

25. Composé selon la revendication 1 où A', L et L¹ suivent l'une des combinaisons suivantes :
A' = isoquinolinyle, L = phényle et L¹ est phényle, pyridinyle, quinolinyle ou isoquinolinyle,
A' = isoquinolinyle, L = pyridinyle et L¹ est phényle, pyridinyle, quinolinyle ou isoquinolinyle,
A' = quinolinyle, L = phényle et L¹ est phényle, pyridinyle, quinolinyle ou isoquinolinyle,
A' = quinolinyle, L = pyridinyle et L¹ est phényle, pyridinyle, quinolinyle ou isoquinolinyle,
A' = quinolinyle substitué, L = phényle et L¹ est phényle, pyridinyle, quinolinyle ou isoquinolinyle, ou
A' = quinolinyle substitué, L = pyridinyle et L¹ est phényle, pyridinyle, quinolinyle ou isoquinolinyle.

26. Composé selon la revendication 1 où L¹ est pyridyle.

27. Utilisation d'un composé selon l'une quelconque des revendications 1 à 26 pour la préparation d'un médicament pour le traitement d'une affection ou inhibition de la voie de la raf kinase est nécessaire.

28. Utilisation d'un composé selon l'une quelconque des revendications 1 à 26 pour la préparation d'un médicament pour le traitement des tumeurs et/ou de la croissance des cellules cancéreuses à médiation par la raf kinase.

29. Utilisation selon la revendication 27 ou 28, où ladite affection est un cancer murin, des cancers solides, comme par exemple des carcinomes, par exemple des poumons, du pancréas, de la thyroïde, de la vessie ou du côlon, des troubles myéloïdes, par exemple la leucémie myéloïde, ou des adénomes, par exemple l'adénome villeux du côlon.
